# EUROPEAN PATENT APPLICATION

(11) **EP 4 223 775 A1**
(43) Date of publication of application: **09.08.2023**
(21) Application number: 21873808.6
(22) Date of filing: 23.02.2021
(51) Int. Cl.: C07K 16/28, C07K 14/21, C07K 1/113, C07K 19/00, A61K 47/68, A61K 39/395, C12N 15/13

(54) **HUMANIZED ANTI-CD22 RECOMBINANT IMMUNOTOXIN AND APPLICATION THEREOF**

(30) Priority: 29.09.2020 CN 202011048723
(71) Applicant: Kunming Sinoway Natural Pharmaceuticals Co., Ltd., Kunming, Yunnan 650217 (CN)
(72) Inventor: HAO, Zhenping, Kunming, Yunnan 650217 (CN); ZHU, Jianwei, Kunming, Yunnan 650217 (CN); XU, Li, Kunming, Yunnan 650217 (CN)
(74) Representative: Schiweck Weinzierl Koch Patentanwälte Partnerschaft mbB
(86) International application number: PCT/CN2021/077461
(87) International publication number: WO 2022/068141

(57) **Abstract**

Provided is a humanized anti-CD22 recombinant immunotoxin, which has reduced immunogenicity and maintains the CD22 binding affinity and corresponding biological activity before humanization transformation, and is used for preparing a drug for treating CD22-related B cell malignant tumors.

## Description

The present application claims the priority benefit of Chinese Patent Application No. CN202011048723.0 filed on 29 September 2020, which is hereby incorporated by reference in its entirety.

### TECHNICAL FIELD

The present invention relates to the field of biomedicines, in particular to a recombinant immunotoxin of humanized anti-CD22 antibody fragment fused with truncated pseudomonas exotoxin A and application thereof.

### BACKGROUND OF THE INVENTION

CD22 (Siglec-2) is a sialic acid-binding immunoglobulin-like lectin (Siglec) receptor, that binds specifically to sialic acid (Sia)-containing glycans, facilitating cell adhesion and/or cell signaling ^{[1]}. CD22 expression is restricted to B cells and plays a critical role in establishing a baseline level of B-cell inhibition, and thus is a critical determinant of homeostasis in humoral immunity. CD22 is expressed in neoplastic cells in various proportions of B-cell malignancies, including B-lymphoblastic leukemia/lymphomas and mature B-cell leukemia/lymphomas ^{[2]}. The expression of CD22 can be particularly strong in hairy cell leukemia (HCL) and prolymphocytic leukemia.

Hairy cell leukemia (HCL), first described by Bouroncle and colleagues in 1958, is a chronic malignancy of mature neoplastic B cells with a characteristic serrated cytoplasmic border ^{[3,4]}. HCL accounts for 2% of all leukemias in the USA (500-800 new cases in the United States per year ^{[5]}) and is characterized by pancytopenia, and splenomegaly. Purine analogues (cladribine or pentostatin) are the standard of care for initial treatment for HCL and are associated with durable remissions that last for years; however, many patients relapse and require additional therapy^{[6]}. Subsequent treatment is usually with a revised regimen of purine analogues, although treatment efficacy is reduced, patients have shorter remissions and are ultimately refractory to treatment^{[7]}. Moreover, purine analogues have been associated with neurotoxicity ^{[8]} and are very immunosuppressive, which may increase the risk of opportunistic infections ^{[4]}.

Immunotoxins have been proven to be effective therapeutics for hairy cell leukemia. Immunotoxins are antibody-conjugated therapeutics that target and kill cancer cells using a potent cytotoxic payload, such as bacterial toxins ^{[9]}, plant-derived toxins ^{[10]}, and synthetic chemicals ^{[11]}. The first generation of the anti-CD22 immunotoxin was developed at the National Cancer Institute in the late 1990s, reported by the name of BL22 (RFB4(dsFv)-PE38, or CAT-3888). It employed a murine anti-CD22 antibody fused to a Pseudomonas exotoxin A (PE38), which is a 38 kDa fragment of Pseudomonas exotoxin A. CAT-3888 entered a phase I trial and reported remissions for leukemia in 2001 ^{[12-16]}. However, CAT-3888 was succeeded by moxetumomab pasudotox (HA22, or CAT-8015), an updated immunotoxin comprising modifications in the PE38 and the anti-CD22 antibody fragment. HA22 made changes to three amino acids in the antibody fragment, comparing to CAT-3888, to increase the binding affinity for the target molecule.

HA22 is composed of the Fv fragment of a murine anti-CD22 monoclonal antibody fused to PE38. Mechanistically, the Fv portion of HA22 binds to CD22, which is a cell surface receptor expressed on a variety of malignant B cells, thereby delivering the toxin moiety PE38 directly to tumor cells. Once internalized, PE38 catalyzes the ADP ribosylation of the diphthamide residue in elongation factor-2 (EF-2), resulting in the rapid fall in levels of the anti-apoptotic protein myeloid cell leukemia 1 (Mcl-1), leading to apoptotic cell death ^{[17,18]}. HA22 was approved by the U.S. FDA in 2018 for the treatment of adults with relapsed or refractory hairy cell leukemia who received at least two prior systemic therapies, including treatment with a purine nucleoside analogue.

The early immunotoxin designs lacked a sufficient therapeutic window to warrant further clinical development, and among which, the inherent immunogenicity and rapidly development of antidrug antibodies was a key factor ^{[19]}. The targeting portion contained in HA22, i.e., the Fv portion of the murine anti-CD22 monoclonal antibody has certain immunogenicity in human since it is murine derived, and thereby causes the generation of undesirable neutralizing anti-drug antibodies (ADAs), and adversely influences pharmacokinetics profiles, including influencing activity of the drug at given dosages, accelerating clearance of the drug in vivo, and limiting times and effectiveness of repeated dosing.

### SUMMARY OF THE INVENTION

In order to solve the above technical problems, an object of the present invention is to provide a novel recombinant immunotoxin based on pseudomonas exotoxin A. In the recombinant immunotoxin, the pseudomonas exotoxin A is fused with a humanized anti-CD22 monoclonal antibody fragment which will have reduced immunogenicity and retain the affinity for the target CD22, compared with the original murine-derived anti-CD22 monoclonal antibody fragment; at the same time, the novel recombinant immunotoxin formed will have a strong immunotoxin efficacy, e.g., growth inhibitory and apoptotic effects on CD22 expressing tumor cells.

A further object of the present invention is to provide the use of the novel recombinant immunotoxin.

Technical solutions of the present invention are as follows.

In one aspect, the present disclosure provides a humanized anti-CD22 recombinant immunotoxin which is a polypeptide molecule comprising two polypeptide chains as follows:
(1) a first polypeptide chain, comprising a light chain variable region (V_{L}) of an anti-CD22 antibody, wherein the light chain variable region (V_{L}) comprises an amino acid sequence as shown in SEQ ID NO. 16 or SEQ ID NO. 18 or an amino acid sequence homologue thereof having at least 75% sequence identity to the amino acid sequence as shown in SEQ ID NO. 16 or SEQ ID NO. 18;
(2) a second polypeptide chain, comprising a heavy chain variable region (V_{H}) of an anti-CD22 antibody and a cytotoxin directly or indirectly linked to the heavy chain variable region (V_{H}), wherein the heavy chain variable region (V_{H}) comprises an amino acid sequence as shown in SEQ ID NO. 3 or SEQ ID NO. 4 or an amino acid sequence homologue thereof having at least 75% identity to the amino acid sequence as shown in SEQ ID NO. 3 or SEQ ID NO. 4.

As used herein, the "amino acid sequence homologue" refers to an amino acid sequence derived from a corresponding amino acid sequence but having one or more amino acid substitutions, additions, or deletions relative thereto.

In the first polypeptide chain, preferably, the amino acid sequence homologue has at least 80%, at least 85%, at least 90%, at least 91%, at least 92%, at least 93%, at least 94%, at least 95%, at least 96%, at least 97%, at least 98%, or at least 99% sequence identity to the amino acid sequence as shown in SEQ ID NO. 16 or SEQ ID NO. 18. Further preferably, the amino acid sequence homologue comprises the amino acid sequences as shown in SEQ ID NO. 31, SEQ ID NO. 32 and SEQ ID NO. 33, and has the same amino acid residues as those in SEQ ID NO. 16 or SEQ ID NO. 18 at positions 3, 5, 36, 37, 47, 48, 49, 50, 65, 67, 69, 70 and 72 corresponding to the amino acid sequence as shown in SEQ ID NO. 14 (the amino acid positions are numbered according to the amino acid sequence as shown in SEQ ID NO. 14), but is not the amino acid sequence as shown in SEQ ID NO. 14.

In the second polypeptide chain, preferably, the amino acid sequence homologue has at least 80%, at least 85%, at least 90%, at least 91%, at least 92%, at least 93%, at least 94%, at least 95%, at least 96%, at least 97%, at least 98%, or at least 99% sequence identity to the amino acid sequence as shown in SEQ ID NO. 3 or SEQ ID NO. 4. Further preferably, the amino acid sequence homologue comprises the amino acid sequences as shown in SEQ ID NO. 28, SEQ ID NO. 29 and SEQ ID NO. 30, and has the same amino acid residues as those in SEQ ID NO. 3 or SEQ ID NO. 4 at positions 3, 48, 49, 50, 69, 71, 73, 75, and 80 corresponding to the amino acid sequence as shown in SEQ ID NO. 2 (the amino acid positions are numbered according to the amino acid sequence as shown in SEQ ID NO. 2), but is not the amino acid sequence as shown in SEQ ID NO. 2.

Preferably, in the humanized anti-CD22 recombinant immunotoxin according to the present invention, the light chain variable region (V_{L}) in the first polypeptide chain comprises the amino acid sequence as shown in SEQ ID NO. 16 or the amino acid sequence homologue thereof; and the heavy chain variable region (V_{H}) in the second polypeptide chain comprises the amino acid sequence as shown in SEQ ID NO. 3 or the amino acid sequence homologue thereof.

Preferably, in the humanized anti-CD22 recombinant immunotoxin according to the present invention, the light chain variable region (V_{L}) in the first polypeptide chain comprises the amino acid sequence as shown in SEQ ID NO. 18 or the amino acid sequence homologue thereof; and the heavy chain variable region (V_{H}) in the second polypeptide chain comprises the amino acid sequence as shown in SEQ ID NO. 3 or the amino acid sequence homologue thereof.

Preferably, in the humanized anti-CD22 recombinant immunotoxin according to the present invention, the light chain variable region (V_{L}) in the first polypeptide chain comprises the amino acid sequence as shown in SEQ ID NO. 16 or the amino acid sequence homologue thereof; and the heavy chain variable region (V_{H}) in the second polypeptide chain comprises the amino acid sequence as shown in SEQ ID NO. 4 or the amino acid sequence homologue thereof.

Preferably, in the humanized anti-CD22 recombinant immunotoxin according to the present invention, the light chain variable region (V_{L}) in the first polypeptide chain and the heavy chain variable region (V_{H}) in the second polypeptide chain are covalently linked, e.g., via a disulfide bond.

Preferably, in the humanized anti-CD22 recombinant immunotoxin according to the present invention, the cytotoxin in the second polypeptide chain is pseudomonas exotoxin A or a mutant or fragment of the pseudomonas exotoxin A with cytotoxicity retained.

Natural occurring pseudomonas exotoxin A (PE) is a bacterial toxin secreted by *Pseudomonas aeruginosa.* It is a monomeric protein with a molecular weight of 66 kD. PE molecule can be modified, or part of its sequence can be removed, to reduce or eliminate nonspecific binding of the toxin while maintaining cytotoxicity. For example, a mutant of pseudomonas exotoxin A is a modified pseudomonas exotoxin A that retains cytotoxicity, and the modification may be conservative modification. For example, a mutant has at least 75%, preferably at least 80%, further preferably at least 90%, 91%, 92%, 93%, 94%, 95% amino acid sequence identity to unmodified natural PE. Also, for example, a fragment of pseudomonas exotoxin A is a truncated form of pseudomonas exotoxin A that retains cytotoxicity. Preferably, the mutant or fragment of pseudomonas exotoxin A according to the present invention is, such as, PE40, PE38, PE35, PE24, mPE24, or T19, T20, M11^{[21, 22, 23, 24, 25]}. According to one particular embodiment of the present invention, the fragment is PE38 with amino acid sequence as shown in SEQ ID NO. 1.

Preferably, in the second polypeptide chain, the cytotoxin is fused to the C-terminus of the heavy chain variable region (V_{H}) of the anti-CD22 antibody. For example, the N-terminus of the cytotoxin is fused, directly or via a linker, to the C-terminus of the heavy chain variable region (V_{H}). The linker can form covalent bonds with the heavy chain variable region (V_{H}) and with the cytotoxin respectively, without affecting the function of each functional component in the humanized anti-CD22 recombinant immunotoxin. Linkers suitable are known in the art, such as linear or branched carbon linkers, heterocyclic carbon linkers, and peptide linkers. In the humanized anti-CD22 recombinant immunotoxin according to the present invention, preferably, no linker is used, or a peptide linker i.e. a linking peptide is used, such as a flexible linking peptide comprising one or more (GGGGS) amino acid sequences.

In the humanized anti-CD22 recombinant immunotoxin according to the present invention, the first polypeptide chain and the second polypeptide chain can constitute a form of Fab, that is, the first polypeptide chain further comprises a kappa light chain constant region, preferably a human kappa light chain constant region fused to the C-terminus of the light chain variable region (V_{L}); alternatively, the second polypeptide chain further comprises a heavy chain constant region CH 1, preferably a human heavy chain constant region CH1 fused to the C-terminus of the heavy chain variable region (V_{H}).

According to one particular embodiment of the present invention, in the humanized anti-CD22 recombinant immunotoxin according to the present invention, the first polypeptide chain comprises the amino acid sequence as shown in SEQ ID NO. 16 or the amino acid sequence homologue thereof, and the second polypeptide chain comprises the amino acid sequence as shown in SEQ ID NO. 9 or the amino acid sequence homolog thereof.

Alternatively, the first polypeptide chain comprises the amino acid sequence as shown in SEQ ID NO. 18 or the amino acid sequence homologue thereof, and the second polypeptide chain comprises the amino acid sequence as shown in SEQ ID NO. 9 or the amino acid sequence homologue thereof.

Alternatively, the first polypeptide chain comprises the amino acid sequence as shown in SEQ ID NO. 16 or the amino acid sequence homologue thereof, and the second polypeptide chain comprises the amino acid sequence as shown in SEQ ID NO. 10 or the amino acid sequence homologue thereof.

In another aspect, the present disclosure provides a nucleic acid molecule comprising a nucleotide sequence encoding the first polypeptide chain in the humanized anti-CD22 recombinant immunotoxin according to the present invention and/or a nucleotide sequence encoding the second polypeptide chain in the humanized anti-CD22 recombinant immunotoxin according to the present invention. The nucleic acid molecule according to the present invention may be a single nucleotide sequence encoding the first polypeptide chain or the second polypeptide chain; or, the nucleic acid molecule according to the present invention may be a single nucleotide sequence encoding both the first polypeptide chain and the second polypeptide chain. Alternatively, the nucleic acid molecule according to the present invention may be a combination of two nucleotide sequences, encoding the first polypeptide chain and the second polypeptide chain, respectively.

In yet another aspect, the present disclosure provides a vector comprising the nucleic acid molecule according to the present invention. The vector can be a eukaryotic expression vector, a prokaryotic expression vector, an artificial chromosome, a phage vector, or the like. According to one particular embodiment of the present invention, the vector is a prokaryotic expression vector, such as an expression plasmid comprising the nucleic acid molecule.

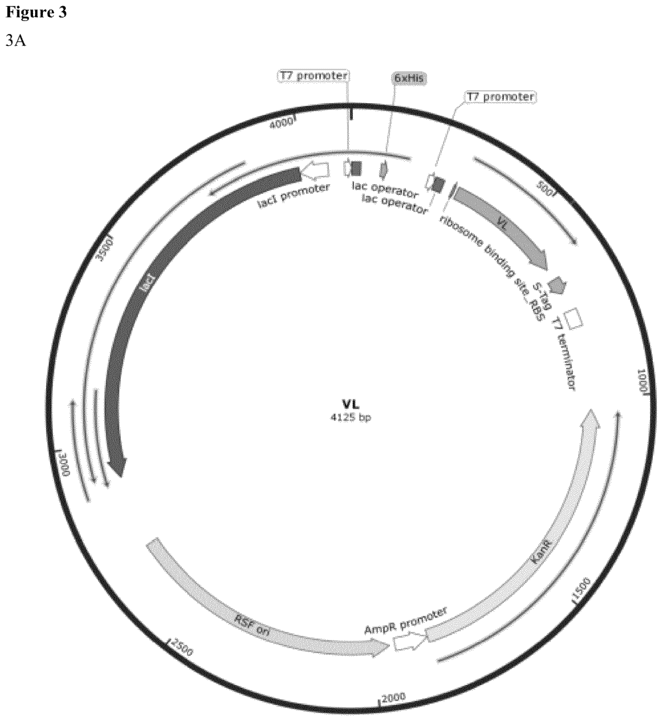

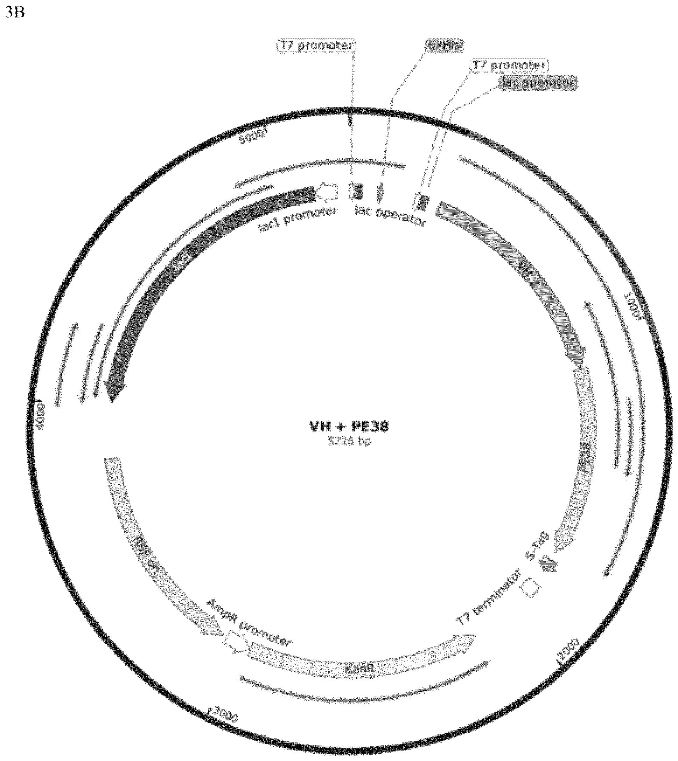

Maps of plasmids according to one particular embodiment of the present invention containing the nucleotide sequences encoding the first polypeptide chain and the second polypeptide chain are shown in FIG. 3.

In yet another aspect, the present disclosure provides a host cell. The host cell is transformed or transfected with the nucleic acid molecule and/or the vector according to the present invention, and thus contains the nucleic acid molecule and/or vector of the present invention, for preservation or expression. The host cell can be any prokaryotic or eukaryotic cell, such as a bacterial or insect, fungal, plant or animal cell. According to one particular embodiment of the present invention, the host cell is a prokaryotic cell, such as E. coli cells.

According to the disclosure of the present invention, the humanized anti-CD22 recombinant immunotoxin, the nucleic acid molecule, the vector and/or the host cell according to the present invention can be obtained by utilizing any conventional techniques known in the art. For example, the first polypeptide chain and the second polypeptide chain can be expressed in a host cell with the nucleic acid molecule or vector according to the present invention, and then a heterodimer, in which the light chain variable region (V_{L}) in the first polypeptide chain is linked to the heavy chain variable region (V_{H}) in the second polypeptide chain via a disulfide bond, can be formed through recovery and in vitro refolding of the chains.

The humanized anti-CD22 recombinant immunotoxin, the nucleic acid molecule, the vector and/or the host cell according to the present invention can be contained in a composition, for example, a pharmaceutical composition, more particularly in a pharmaceutical preparation, to be used for various purposes as actually needed.

Thus, in still a further aspect, the present disclosure also provides a composition comprising the humanized anti-CD22 recombinant immunotoxin, the nucleic acid molecule, the vector and/or the host cell according to the present invention. Preferably, the composition is a pharmaceutical composition which optionally comprises a pharmaceutically acceptable excipient.

The present disclosure further provides following related uses of the subject matters described above based on the humanized anti-CD22 recombinant immunotoxin which is capable of binding to target CD22, especially human CD22, a B cell surface marker.

Specifically, in one aspect, the present disclosure provides use of the humanized anti-CD22 recombinant immunotoxin, the nucleic acid molecule, the vector, the host cell or the composition in the manufacture of a medicament for the treatment of a CD22-related B cell malignant tumor.

Preferably, the CD22-related B-cell malignant tumor is a B-cell malignant tumor characterized by high CD22 expression, such as a lymphoma or leukemia with high CD22 expression. Preferably, the lymphoma is non-Hodgkin's lymphoma, small lymphocytic lymphoma or mantle cell lymphoma; and the leukemia is chronic lymphocytic leukemia, hairy cell leukemia or acute lymphocytic leukemia.

In another aspect, the present disclosure provides a method for treating a CD22-related B-cell malignant tumor, comprising administering to a subject in need thereof the humanized anti-CD22 recombinant immunotoxin, the nucleic acid molecule, the vector and/or the host cell. The subject is a mammal, including human or non-human primate, and a domestic, livestock or laboratory mammal such as a dog, a cat, a cow, a pig, a sheep, a horse, a murine, and a rabbit. Preferably, the subject is a human.

The humanized V_{H} and V_{L} according to the present invention can also form a separate dsFv antibody, so in yet another aspect, the present disclosure provides a humanized anti-CD22 antibody or antibody fragment, the antibody or antibody fragment comprising a heavy chain variable region (V_{H}) and a light chain variable region (V_{L}), wherein the heavy chain variable region (V_{H}) comprises an amino acid sequence as shown in SEQ ID NO. 3 or SEQ ID NO. 4 or an amino acid sequence homologue thereof having at least 75% identity to the amino acid sequence as shown in SEQ ID NO. 3 or SEQ ID NO. 4, and the light chain variable region (V_{L}) comprises an amino acid sequence as shown in SEQ ID NO. 16 or SEQ ID NO. 18 or an amino acid sequence homologue thereof having at least 75% identity to the amino acid sequence as shown in SEQ ID NO. 16 or SEQ ID NO. 18.

In the light chain variable region (V_{L}), preferably, the amino acid sequence homologue has at least 80%, at least 85%, at least 90%, at least 91%, at least 92%, at least 93%, at least 94%, at least 95%, at least 96%, at least 97%, at least 98%, or at least 99% sequence identity to the amino acid sequence as shown in SEQ ID NO. 16 or SEQ ID NO. 18. Further preferably, the amino acid sequence homologue comprises the amino acid sequences as shown in SEQ ID NO. 31, SEQ ID NO. 32 and SEQ ID NO. 33, and has the same amino acid residues as those in SEQ ID NO. 16 or SEQ ID NO. 18 at positions 3, 5, 36, 37, 47, 48, 49, 50, 65, 67, 69, 70 and 72 corresponding to the amino acid sequence as shown in SEQ ID NO. 14 (the amino acid positions are numbered according to the amino acid sequence as shown in SEQ ID NO. 14), but is not the amino acid sequence as shown in SEQ ID NO. 14.

In the heavy chain variable region (V_{H}), preferably, the amino acid sequence homologue has at least 80%, at least 85%, at least 90%, at least 91%, at least 92%, at least 93%, at least 94%, at least 95%, at least 96%, at least 97%, at least 98%, or at least 99% sequence identity to the amino acid sequence as shown in SEQ ID NO. 3 or SEQ ID NO. 4. Further preferably, the amino acid sequence homologue comprises the amino acid sequences as shown in SEQ ID NO. 28, SEQ ID NO. 29 and SEQ ID NO. 30, and has the same amino acid residues as those in SEQ ID NO. 3 or SEQ ID NO. 4 at positions 3, 48, 49, 50, 69, 71, 73, 75, and 80 corresponding to the amino acid sequence as shown in SEQ ID NO. 2 (the amino acid positions are numbered according to the amino acid sequence as shown in SEQ ID NO. 2), but is not the amino acid sequence as shown in SEQ ID NO. 2.

Preferably, in the humanized anti-CD22 antibody or antibody fragment according to the present invention, the light chain variable region (V_{L}) comprises the amino acid sequence as shown in SEQ ID NO. 16 or the amino acid sequence homologue thereof, and the heavy chain variable region (V_{H}) comprises the amino acid sequence as shown in SEQ ID NO. 3 or the amino acid sequence homologue thereof.

Preferably, in the humanized anti-CD22 antibody or antibody fragment according to the present invention, the light chain variable region (V_{L}) comprises the amino acid sequence as shown in SEQ ID NO. 18 or the amino acid sequence homologue thereof; and the heavy chain variable region (V_{H}) comprises the amino acid sequence as shown in SEQ ID NO. 3 or the amino acid sequence homologue thereof.

Preferably, in the humanized anti-CD22 antibody or antibody fragment according to the present invention, the light chain variable region (V_{L}) comprises the amino acid sequence as shown in SEQ ID NO. 16 or the amino acid sequence homologue thereof; and the heavy chain variable region (V_{H}) comprises the amino acid sequence as shown in SEQ ID NO. 4 or the amino acid sequence homologue thereof.

Preferably, the humanized anti-CD22 antibody according to the present invention is a monoclonal antibody, which further comprises a human heavy chain constant region (CH) and a light chain constant region (CL); preferably, the monoclonal antibody comprises a heavy chain (HC) and a light chain (LC).

Depending on the heavy chain constant region sequence of the monoclonal antibody, the antibody can be of IgA, IgD, IgE, IgG or IgM class, even further of a subclass (isotype) such as IgG1, IgG2, IgG3, or IgG4. Depending on the light chain constant region sequence of the monoclonal antibody, the antibody can be of kappa type or lambda type.

Preferably, the antibody fragment according to the present invention is a monoclonal antibody fragment, preferably Fab, Fab', F(ab')2, scFv, dsFv or any other fragment of the antibody that retains the ability to recognize or bind to CD22. Depending on the context, "antibody fragment" in the present disclosure can be used interchangeably with "antibody".

More preferably, the antibody fragment according to the present invention is a dsFv fragment of a monoclonal antibody, that is, a disulfide-stabilized Fv fragment in which the heavy chain variable region and the light chain variable region are linked via a disulfide bond. According to one particular embodiment of the present invention, the dsFv fragment is a heterodimer consisting of a heavy chain variable region and a light chain variable region linked via a disulfide bond.

In yet another aspect, the present disclosure also provides use of the humanized anti-CD22 antibody in the preparation of a recombinant immunotoxin. For example, any chain of the humanized anti-CD22 antibody can be linked to a cytotoxin of the present invention to prepare a recombinant immunotoxin.

The present invention provides a new humanized anti-CD22 recombinant immunotoxin composed of a humanized anti-CD22 monoclonal antibody fragment fused with truncated pseudomonas exotoxin A, specifically a heterodimer composed of a heavy chain variable region and a light chain variable region linked via a disulfide bond, wherein the heavy chain variable region is fused with the truncated pseudomonas exotoxin A.

Compared with the recombinant immunotoxin before humanization engineering, the humanized anti-CD22 recombinant immunotoxin according to the present invention is obtained through a specific humanization strategy, so better resembles those of human counterparts, and thus potentially reduce the immunogenicity of the Fv portion of the immunotoxin in human. The reduced immunogenicity could prevent the generation of undesirable neutralizing anti-drug antibodies (ADAs) and contribute to better pharmacokinetics profiles when used in clinics.

Moreover, the humanized anti-CD22 recombinant immunotoxin according to the present invention maintains the best affinity for the target CD22 and the corresponding biological activity while having maximum humanization. Compared with the recombinant immunotoxin before humanization engineering, the humanized anti-CD22 recombinant immunotoxin of the present invention maintains the binding affinity for receptor CD22 and the cell growth inhibitory and apoptotic effects on effector cells. In addition, the humanized anti-CD22 recombinant immunotoxin exhibits comparable pharmacokinetics and tumor suppression efficacy to that one before the engineering.

### BRIEF DESCRIPTION OF THE DRAWINGS

Embodiments of the present invention are described in detail below with reference to the attached figures, in which:
Figure 1 shows the schematic diagram of the humanized anti-CD22 recombinant immunotoxin according to the present invention.
Figure 2 shows the alignment results of different versions of humanized sequences performed in the present disclosure, in which panel 2A: VH; panel 2B: VL.
Figure 3 shows the expression plasmid maps of the humanized anti-CD22 recombinant immunotoxin according to the present invention, in which panel 3A: the expression plasmid of the first polypeptide chain; panel 3B: the expression plasmid of the second polypeptide chain.
Figure 4 shows the PK profiles of anti-CD22 recombinant immunotoxins, in which panel 4A: HA22; panel 4B: H1L2.
Figure 5 shows the tumor suppression efficacy of anti-CD22 recombinant immunotoxins.

### DETAILED DESCRIPTION OF PREFERRED EMBODIMENTS

As introduced in the present disclosure, a humanization strategy was employed to optimize the Fv region of the anti-CD22 portion of HA22 to better resemble those of human counterparts, and thus potentially reduce the immunogenicity of the Fv portion of the immunotoxin in human. The reduced immunogenicity could prevent the generation of undesirable neutralizing anti-drug antibody (ADA) and contribute to better pharmacokinetics profiles when used in clinics.

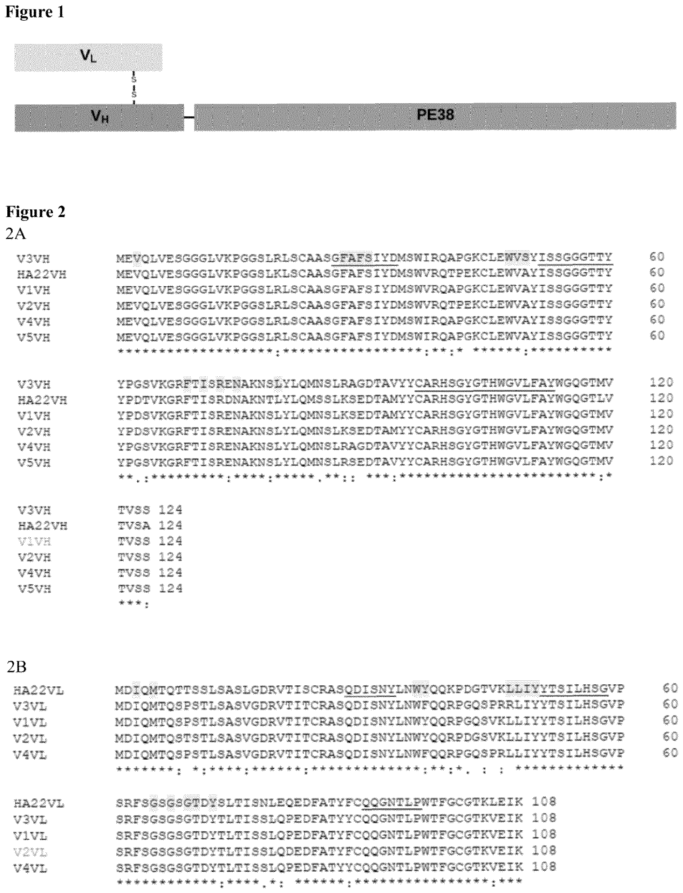

Accordingly, the present disclosure provides a humanized anti-CD22 recombinant immunotoxin, comprising a humanized anti-CD22 monoclonal antibody fragment fused with truncated pseudomonas exotoxin A. Specifically, the humanized anti-CD22 recombinant immunotoxin is a heterodimer comprised of a kappa light chain V region (a first polypeptide chain) and a heavy chain V region linked by one disulfide bond, wherein the heavy chain V region is fused with a truncated pseudomonas exotoxin A (a second polypeptide chain). The schematic diagram of the recombinant immunotoxin is shown in Figure 1, in which the truncated pseudomonas exotoxin A is illustratively PE38.

According to the present invention, the humanized anti-CD22 recombinant immunotoxin binds to CD22 receptor on the malignant B-cell surface to deliver the toxin moiety e.g., PE38 directly to tumor cells. Upon internalization, the toxin part catalyzes the ADP ribosylation of the diphthamide residue in elongation factor-2 (EF-2), resulting in the rapid fall in levels of the anti-apoptotic protein myeloid cell leukemia 1 (Mcl-1), leading to apoptotic cell death.

The humanized anti-CD22 recombinant immunotoxin according to the present invention can be produced in, for example, an enhanced BL21 derivative strain (T7 Express) containing an expression plasmid harboring nucleotide sequence coding for the first polypeptide chain and an expression plasmid harboring nucleotide sequence coding for the second polypeptide chain. The resulting strain is resistant to kanamycin and the production of the humanized anti-CD22 recombinant immunotoxin is induced by Isopropyl β-d-1-thiogalactopyranoside (IPTG). The product is expressed as inclusion bodies. The inclusion bodies can be recovered and then refolded in vitro to form a heterodimer. The refolded protein can be then purified by hydrophobic interaction chromatography followed by anion exchange chromatography. The purified protein is finally concentrated and diafiltered into a buffer prepared for storage.

The humanized anti-CD22 recombinant immunotoxin according to the present invention is a humanized version of HA22, and therefore is expected to interact with CD22 expressing cells via identical/similar mechanism of action(s). In vitro and in vivo assays were designed to assess immunotoxin molecules' CD22 binding affinity, cytotoxicity in CD22-expressing cells, pharmacokinetics and tumor suppression efficacy. HA22 used in the studies was manufactured in-house using the same production process as that of the recombinant immunotoxin.

In the present disclosure, the interaction between the antibody fragment of the humanized anti-CD22 recombinant immunotoxin of the present invention and the CD22 receptor was determined in in vitro binding assay; and the growth inhibition and apoptotic cell death effects of the humanized anti-CD22 recombinant immunotoxin were examined in cell-based assay. Further, the pharmacokinetic and tumor suppression efficacy of the humanized anti-CD22 recombinant immunotoxin of the present invention were determined in in vivo studies performed.

Sequences used in the present disclosure are as follows:
SEQ ID NO. 1: PE38 polypeptide sequence 
KASGGP EGGSLAALTA HQACHLPLET FTRHRQPRGW EQLEQCGYPV QRLVALYLAA
RLSWNQVDQV IRNALASPGS GGDLGEAIRE QPEQARLALT LAAAESERFV
RQGTGNDEAG AANGPADSGD ALLERNYPTG AEFLGDGGDV SFSTRGTQNW
TVERLLQAHR QLEERGYVFV GYHGTFLEAA QSIVFGGVRA RSQDLDAIWR
 
GFYIAGDPAL AYGYAQDQEP DARGRIRNGA LLRVYVPRSS LPGFYRTSLT
LAAPEAAGEV ERLIGHPLPL RLDAITGPEE EGGRLETILG WPLAERTVVI PSAIPTDPRN
VGGDLDPSSI PDKEQAISAL PDYASQPGKP PREDLK
SEQ ID NO. 2: HA22, VH polypeptide sequence (HA22 V_{H}) 
MEVQLVESGG GLVKPGGSLK LSCAASGFAF SIYDMSWVRQ TPEKCLEWVA
YISSGGGTTY YPDTVKGRFT ISRDNAKNTL YLQMSSLKSE DTAMYYCARH
SGYGTHWGVL FAYWGQGTLV TVSA
SEQ ID NO. 3: Version 1 VH polypeptide sequence (V_{H} ver 1) 
MEVQLVESGG GLVKPGGSLR LSCAASGFAF SIYDMSWVRQ APGKCLEWVA
YISSGGGTTY YPDSVKGRFT ISRENAKNSL YLQMNSLKSE DTAMYYCARH
SGYGTHWGVL FAYWGQGTMV TVSS
SEQ ID NO. 4: Version 2 VH polypeptide sequence (V_{H} ver 2) 
MEVQLVESGG GLVKPGGSLR LSCAASGFAF SIYDMSWVRQ TPEKCLEWVA
YISSGGGTTY YPDSVKGRFT ISRENAKNSL YLQMNSLKSE DTAMYYCARH
SGYGTHWGVL FAYWGQGTMV TVSS
SEQ ID NO. 5: Version 3 VH polypeptide sequence (V_{H} ver 3) 
MEVQLVESGG GLVKPGGSLR LSCAASGFAF SIYDMSWIRQ APGKCLEWVS
YISSGGGTTY YPGSVKGRFT ISRENAKNSL YLQMNSLRAG DTAVYYCARH
SGYGTHWGVL FAYWGQGTMV TVSS
SEQ ID NO. 6: Version 4 VH polypeptide sequence (V_{H} ver 4) 
MEVQLVESGG GLVKPGGSLR LSCAASGFAF SIYDMSWIRQ APGKCLEWVA
YISSGGGTTY YPGSVKGRFT ISRENAKNSL YLQMNSLRAG DTAVYYCARH
SGYGTHWGVL FAYWGQGTMV TVSS
SEQ ID NO. 7: Version 5 VH polypeptide sequence (V_{H} ver 5) MEVQLVESGG GLVKPGGSLR LSCAASGFAF SIYDMSWIRQ APGKCLEWVA 
YISSGGGTTY YPGSVKGRFT ISRENAKNSL YLQMNSLRSE DTAVYYCARH
SGYGTHWGVL FAYWGQGTMV TVSS
SEQ ID NO. 8: HA22 VH-PE38 polypeptide sequence 
MEVQLVESGG GLVKPGGSLK LSCAASGFAF SIYDMSWVRQ TPEKCLEWVA
YISSGGGTTY YPDTVKGRFT ISRDNAKNTL YLQMSSLKSE DTAMYYCARH
SGYGTHWGVL FAYWGQGTLV TVSAKASGGP EGGSLAALTA HQACHLPLET
FTRHRQPRGW EQLEQCGYPV QRLVALYLAA RLSWNQVDQV IRNALASPGS
GDLGEAIRE QPEQARLALT LAAAESERFV RQGTGNDEAG AANGPADSGD
ALLERNYPTG AEFLGDGGDV SFSTRGTQNW TVERLLQAHR QLEERGYVFV
GYHGTFLEAA QSIVFGGVRA RSQDLDAIWR GFYIAGDPAL AYGYAQDQEP
DARGRIRNGA LLRVYVPRSS LPGFYRTSLT LAAPEAAGEV ERLIGHPLPL
RLDAITGPEE EGGRLETILG WPLAERTVVI PSAIPTDPRN VGGDLDPSSI PDKEQAISAL
PDYASQPGKP PREDLK
SEQ ID NO. 9: Version 1 VH-PE38 polypeptide sequence 
MEVQLVESGG GLVKPGGSLR LSCAASGFAF SIYDMSWVRQ APGKCLEWVA
YISSGGGTTY YPDSVKGRFT ISRENAKNSL YLQMNSLKSE DTAMYYCARH
SGYGTHWGVL FAYWGQGTMV TVSSKASGGP EGGSLAALTA HQACHLPLET
FTRHRQPRGW EQLEQCGYPV QRLVALYLAA RLSWNQVDQV IRNALASPGS
GDLGEAIRE QPEQARLALT LAAAESERFV RQGTGNDEAG AANGPADSGD
ALLERNYPTG AEFLGDGGDV SFSTRGTQNW TVERLLQAHR QLEERGYVFV
GYHGTFLEAA QSIVFGGVRA RSQDLDAIWR GFYIAGDPAL AYGYAQDQEP
DARGRIRNGA LLRVYVPRSS LPGFYRTSLT LAAPEAAGEV ERLIGHPLPL
RLDAITGPEE EGGRLETILG WPLAERTVVI PSAIPTDPRN VGGDLDPSSI PDKEQAISAL
PDYASQPGKP PREDLK
SEQ ID NO. 10: Version 2 VH-PE38 polypeptide sequence 
MEVQLVESGG GLVKPGGSLR LSCAASGFAF SIYDMSWVRQ TPEKCLEWVA
YISSGGGTTY YPDSVKGRFT ISRENAKNSL YLQMNSLKSE DTAMYYCARH
SGYGTHWGVL FAYWGQGTMV TVSSKASGGP EGGSLAALTA HQACHLPLET
 
FTRHRQPRGW EQLEQCGYPV QRLVALYLAA RLSWNQVDQV IRNALASPGS
GGDLGEAIRE QPEQARLALT LAAAESERFV RQGTGNDEAG AANGPADSGD
ALLERNYPTG AEFLGDGGDV SFSTRGTQNW TVERLLQAHR QLEERGYVFV
GYHGTFLEAA QSIVFGGVRA RSQDLDAIWR GFYIAGDPAL AYGYAQDQEP
DARGRIRNGA LLRVYVPRSS LPGFYRTSLT LAAPEAAGEV ERLIGHPLPL
RLDAITGPEE EGGRLETILG WPLAERTVVI PSAIPTDPRN VGGDLDPSSI PDKEQAISAL
PDYASQPGKP PREDLK
SEQ ID NO. 11: Version 3 VH-PE38 polypeptide sequence 
MEVQLVESGG GLVKPGGSLR LSCAASGFAF SIYDMSWIRQ APGKCLEWVS
YISSGGGTTY YPGSVKGRFT ISRENAKNSL YLQMNSLRAG DTAVYYCARH
SGYGTHWGVL FAYWGQGTMV TVSSKASGGP EGGSLAALTA HQACHLPLET
FTRHRQPRGW EQLEQCGYPV QRLVALYLAA RLSWNQVDQV IRNALASPGS
GGDLGEAIRE QPEQARLALT LAAAESERFV RQGTGNDEAG AANGPADSGD
ALLERNYPTG AEFLGDGGDV SFSTRGTQNW TVERLLQAHR QLEERGYVFV
GYHGTFLEAA QSIVFGGVRA RSQDLDAIWR GFYIAGDPAL AYGYAQDQEP
DARGRIRNGA LLRVYVPRSS LPGFYRTSLT LAAPEAAGEV ERLIGHPLPL
RLDAITGPEE EGGRLETILG WPLAERTVVI PSAIPTDPRN VGGDLDPSSI PDKEQAISAL
PDYASQPGKP PREDLK
SEQ ID NO. 12: Version 4 VH-PE38 polypeptide sequence 
MEVQLVESGG GLVKPGGSLR LSCAASGFAF SIYDMSWIRQ APGKCLEWVA
YISSGGGTTY YPGSVKGRFT ISRENAKNSL YLQMNSLRAG DTAVYYCARH
SGYGTHWGVL FAYWGQGTMV TVSSKASGGP EGGSLAALTA HQACHLPLET
FTRHRQPRGW EQLEQCGYPV QRLVALYLAA RLSWNQVDQV IRNALASPGS
GGDLGEAIRE QPEQARLALT LAAAESERFV RQGTGNDEAG AANGPADSGD
ALLERNYPTG AEFLGDGGDV SFSTRGTQNW TVERLLQAHR QLEERGYVFV
GYHGTFLEAA QSIVFGGVRA RSQDLDAIWR GFYIAGDPAL AYGYAQDQEP
DARGRIRNGA LLRVYVPRSS LPGFYRTSLT LAAPEAAGEV ERLIGHPLPL
RLDAITGPEE EGGRLETILG WPLAERTVVI PSAIPTDPRN VGGDLDPSSI PDKEQAISAL
PDYASQPGKP PREDLK
SEQ ID NO. 13: Version 5 VH-PE38 polypeptide sequence 
MEVQLVESGG GLVKPGGSLR LSCAASGFAF SIYDMSWIRQ APGKCLEWVA
YISSGGGTTY YPGSVKGRFT ISRENAKNSL YLQMNSLRSE DTAVYYCARH
SGYGTHWGVL FAYWGQGTMV TVSSKASGGP EGGSLAALTA HQACHLPLET
FTRHRQPRGW EQLEQCGYPV QRLVALYLAA RLSWNQVDQV IRNALASPGS
GGDLGEAIRE QPEQARLALT LAAAESERFV RQGTGNDEAG AANGPADSGD
ALLERNYPTG AEFLGDGGDV SFSTRGTQNW TVERLLQAHR QLEERGYVFV
GYHGTFLEAA QSIVFGGVRA RSQDLDAIWR GFYIAGDPAL AYGYAQDQEP
DARGRIRNGA LLRVYVPRSS LPGFYRTSLT LAAPEAAGEV ERLIGHPLPL
RLDAITGPEE EGGRLETILG WPLAERTVVI PSAIPTDPRN VGGDLDPSSI PDKEQAISAL
PDYASQPGKP PREDLK
SEQ ID NO. 14: HA22, VL polypeptide sequence (HA22 V_{L}) 
MDIQMTQTTS SLSASLGDRV TISCRASQDI SNYLNWYQQK PDGTVKLLIY
YTSILHSGVP SRFSGSGSGT DYSLTISNLE QEDFATYFCQ QGNTLPWTFG CGTKLEIK
SEQ ID NO. 15: Version 1 VL polypeptide sequence (V_{L} ver 1) 
MDIQMTQSPS TLSASVGDRV TITCRASQDI SNYLNWYQQR PGQSVKLLIY
YTSILHSGVP SRFSGSGSGT DYTLTISSLQ PEDFATYFCQ QGNTLPWTFG CGTKVEIK
SEQ ID NO. 16: Version 2 VL polypeptide sequence (V_{L} ver 2) 
MDIQMTQSTS TLSASVGDRV TITCRASQDI SNYLNWYQQR PDGSVKLLIY
YTSILHSGVP SRFSGSGSGT DYTLTISSLQ DEDFATYFCQ QGNTLPWTFG CGTKVEIK
SEQ ID NO. 17: Version 3 VL polypeptide sequence (V_{L} ver 3) 
MDIQMTQSPS TLSASVGDRV TITCRASQDI SNYLNWFQQR PGQSPRRLIY
YTSILHSGVP SRFSGSGSGT DYTLTISSLQ PEDFATYYCQ QGNTLPWTFG CGTKVEIK
SEQ ID NO. 18: Version 4 VL polypeptide sequence (V_{L} ver 4 
[?]
MDIQMTQSPS TLSASVGDRV TITCRASQDI SNYLNWFQQR PGQSPRLLIY
 YTSILHSGVP SRFSGSGSGT DYTLTISSLQ PEDFATYYCQ QGNTLPWTFG CGTKVEIK
SEQ ID NO. 19: Version 1 VH-PE38 DNA sequence 
ATGGAAGTGCAGCTGGTGGAATCCGGCGGAGGCCTGGTCAAGCCCGGCGGCTCTCT
GAGACTGTCCTGCGCTGCTTCTGGCTTCGCCTTCTCCATCTACGACATGTCCTGGGTC
AGACAGGCCCCTGGCAAGTGCCTGGAATGGGTGGCCTACATCTCCTCTGGCGGAGGC
ACCACCTACTACCCTGACTCCGTGAAGGGCAGATTCACCATCTCTAGAGAGAACGCC
AAGAACAGCCTGTACCTGCAGATGAACTCCCTGAAGTCCGAGGACACCGCCATGTAC
TACTGCGCCAGACACTCCGGCTACGGCACACACTGGGGCGTGCTGTTCGCTTACTGG
GGCCAGGGCACCATGGTGACCGTGTCCTCCAAAGCGTCAGGCGGTCCGGAAGGCGG
TTCGCTGGCAGCTCTGACCGCACATCAGGCATGCCACCTGCCGCTGGAAACCTTTAC
CCGCCATCGTCAACCGCGCGGCTGGGAACAGCTGGAACAATGTGGTTATCCGGTGCA
GCGCCTGGTTGCCCTGTACCTGGCCGCACGTCTGAGCTGGAACCAGGTTGATCAAGT
CATTCGTAATGCGCTGGCATCACCGGGCTCGGGCGGTGACCTGGGTGAAGCAATCCG
CGAACAGCCGGAACAAGCGCGCCTGGCCCTGACCCTGGCAGCTGCGGAATCCGAAC
GCTTTGTGCGTCAGGGCACCGGTAATGATGAAGCCGGTGCAGCAAATGGTCCGGCTG
ATTCAGGTGACGCGCTGCTGGAACGCAACTATCCGACGGGCGCCGAATTTCTGGGTG
ATGGCGGTGACGTGAGTTTCTCCACCCGCGGCACGCAGAATTGGACCGTTGAACGTC
TGCTGCAGGCGCATCGCCAACTGGAAGAACGTGGTTATGTTTTTGTCGGCTATCATGG
CACCTTTCTGGAAGCTGCGCAGTCGATTGTCTTTGGCGGTGTGCGTGCACGCAGCCA
GGATCTGGATGCAATTTGGCGCGGCTTCTACATCGCAGGTGATCCGGCTCTGGCGTAT
GGCTACGCTCAGGATCAAGAACCGGATGCGCGTGGCCGCATCCGTAATGGTGCCCTG
CTGCGTGTGTATGTTCCGCGTTCATCGCTGCCGGGTTTTTACCGTACCTCTCTGACCCT
GGCGGCACCGGAAGCTGCCGGCGAAGTGGAACGCCTGATTGGTCACCCGCTGCCGC
TGCGTCTGGATGCAATCACCGGTCCGGAAGAAGGCGGCCGTCTGGAAACGATTC
TGGGTTGGCCGCTGGCCGAACGTACCGTGGTTATTCCGAGCGCAATCCCGACGGACC
CGCGCAATGTTGGCGGTGATCTGGACCCGAGCTCTATTCCGGATAAAGAACAGGCCA
TCTCCGCACTGCCGGACTATGCGTCACAACCGGGCAAACCGCCGCGTGAAGACCTGA
AA
SEQ ID NO. 20: Version 2 VH-PE38 DNA sequence 
ATGGAAGTGCAGCTGGTGGAATCCGGCGGAGGCCTGGTCAAGCCCGGCGGCTCTCT
GAGACTGTCCTGCGCTGCTTCTGGCTTCGCCTTCTCCATCTACGACATGTCCTGGGTC
AGACAGACCCCTGAAAAGTGCCTGGAATGGGTGGCCTACATCTCCTCTGGCGGAGGC
ACCACCTACTACCCTGACTCCGTGAAGGGCAGATTCACCATCTCTAGAGAGAACGCC
AAGAACAGCCTGTACCTGCAGATGAACTCCCTGAAGTCCGAGGACACCGCCATGTAC
TACTGCGCCAGACACTCCGGCTACGGCACACACTGGGGCGTGCTGTTCGCTTACTGG
GGCCAGGGCACCATGGTGACCGTGTCCTCCAAAGCGTCAGGCGGTCCGGAAGGCGG
TTCGCTGGCAGCTCTGACCGCACATCAGGCATGCCACCTGCCGCTGGAAACCTTTAC
CCGCCATCGTCAACCGCGCGGCTGGGAACAGCTGGAACAATGTGGTTATCCGGTGCA
GCGCCTGGTTGCCCTGTACCTGGCCGCACGTCTGAGCTGGAACCAGGTTGATCAAGT
CATTCGTAATGCGCTGGCATCACCGGGCTCGGGCGGTGACCTGGGTGAAGCAATCCG
CGAACAGCCGGAACAAGCGCGCCTGGCCCTGACCCTGGCAGCTGCGGAATCCGAAC
GCTTTGTGCGTCAGGGCACCGGTAATGATGAAGCCGGTGCAGCAAATGGTCCGGCTG
ATTCAGGTGACGCGCTGCTGGAACGCAACTATCCGACGGGCGCCGAATTTCTGGGTG
ATGGCGGTGACGTGAGTTTCTCCACCCGCGGCACGCAGAATTGGACCGTTGAACGTC
TGCTGCAGGCGCATCGCCAACTGGAAGAACGTGGTTATGTTTTTGTCGGCTATCATGG
CACCTTTCTGGAAGCTGCGCAGTCGATTGTCTTTGGCGGTGTGCGTGCACGCAGCCA
GGATCTGGATGCAATTTGGCGCGGCTTCTACATCGCAGGTGATCCGGCTCTGGCGTAT
GGCTACGCTCAGGATCAAGAACCGGATGCGCGTGGCCGCATCCGTAATGGTGCCCTG
CTGCGTGTGTATGTTCCGCGTTCATCGCTGCCGGGTTTTTACCGTACCTCTCTGACCCT
GGCGGCACCGGAAGCTGCCGGCGAAGTGGAACGCCTGATTGGTCACCCGCTGCCGC
TGCGTCTGGATGCAATCACCGGTCCGGAAGAAGGCGGCCGTCTGGAAACGATTC
TGGGTTGGCCGCTGGCCGAACGTACCGTGGTTATTCCGAGCGCAATCCCGACGGACC
CGCGCAATGTTGGCGGTGATCTGGACCCGAGCTCTATTCCGGATAAAGAACAGGCCA
TCTCCGCACTGCCGGACTATGCGTCACAACCGGGCAAACCGCCGCGTGAAGACCTGA
AA
SEQ ID NO. 21: Version 3 VH-PE38 DNA sequence 
ATGGAAGTGCAGCTGGTTGAAAGCGGTGGCGGTCTGGTGAAACCGGGCGGTAGCCT
GCGTCTGAGCTGCGCGGCGAGCGGTTTCGCGTTTAGCATCTACGACATGAGCTGGATT
CGTCAAGCGCCGGGCAAATGCCTGGAGTGGGTGAGCTATATCAGCAGCGGCGGTGGC
 
ACCACCTACTATCCGGGTAGCGTTAAGGGCCGTTTCACCATTAGCCGTGAAAACGCG
AAAAACAGCCTGTACCTGCAGATGAACAGCCTGCGTGCGGGTGATACCGCTGTGTAC
TATTGCGCGCGTCACAGCGGTTACGGCACCCACTGGGGCGTTCTGTTTGCGTATTGGG
GTCAAGGCACCATGGTGACCGTTAGCAGCAAAGCGTCAGGCGGTCCGGAAGGCGGT
TCGCTGGCAGCTCTGACCGCACATCAGGCATGCCACCTGCCGCTGGAAACCTTTACC
CGCCATCGTCAACCGCGCGGCTGGGAACAGCTGGAACAATGTGGTTATCCGGTGCAG
CGCCTGGTTGCCCTGTACCTGGCCGCACGTCTGAGCTGGAACCAGGTTGATCAAGTC
ATTCGTAATGCGCTGGCATCACCGGGCTCGGGCGGTGACCTGGGTGAAGCAATCCGC
GAACAGCCGGAACAAGCGCGCCTGGCCCTGACCCTGGCAGCTGCGGAATCCGAACG
CTTTGTGCGTCAGGGCACCGGTAATGATGAAGCCGGTGCAGCAAATGGTCCGGCTGA
TTCAGGTGACGCGCTGCTGGAACGCAACTATCCGACGGGCGCCGAATTTCTGGGTGA
TGGCGGTGACGTGAGTTTCTCCACCCGCGGCACGCAGAATTGGACCGTTGAACGTCT
GCTGCAGGCGCATCGCCAACTGGAAGAACGTGGTTATGTTTTTGTCGGCTATCATGGC
ACCTTTCTGGAAGCTGCGCAGTCGATTGTCTTTGGCGGTGTGCGTGCACGCAGCCAG
GATCTGGATGCAATTTGGCGCGGCTTCTACATCGCAGGTGATCCGGCTCTGGCGTATG
GCTACGCTCAGGATCAAGAACCGGATGCGCGTGGCCGCATCCGTAATGGTGCCCTGC
TGCGTGTGTATGTTCCGCGTTCATCGCTGCCGGGTTTTTACCGTACCTCTCTGACCCTG
GCGGCACCGGAAGCTGCCGGCGAAGTGGAACGCCTGATTGGTCACCCGCTGCCGCT
GCGTCTGGATGCAATCACCGGTCCGGAAGAAGGCGGCCGTCTGGAAACGATTCT
GGGTTGGCCGCTGGCCGAACGTACCGTGGTTATTCCGAGCGCAATCCCGACGGACCC
GCGCAATGTTGGCGGTGATCTGGACCCGAGCTCTATTCCGGATAAAGAACAGGCCAT
CTCCGCACTGCCGGACTATGCGTCACAACCGGGCAAACCGCCGCGTGAAGACCTGA
AA
SEQ ID NO. 22: Version 4 VH-PE38 DNA sequence 
ATGGAAGTGCAGCTGGTTGAAAGCGGTGGCGGTCTGGTGAAACCGGGCGGTAGCCT
GCGTCTGAGCTGCGCGGCGAGCGGTTTCGCGTTTAGCATCTACGACATGAGCTGGATT
CGTCAAGCGCCGGGCAAATGCCTGGAGTGGGTGGCGTATATCAGCAGCGGCGGTGGC
ACCACCTACTATCCGGGTAGCGTTAAGGGCCGTTTCACCATTAGCCGTGAAAACGCG
AAAAACAGCCTGTACCTGCAGATGAACAGCCTGCGTGCGGGTGATACCGCTGTGTAC
TATTGCGCGCGTCACAGCGGTTACGGCACCCACTGGGGCGTTCTGTTTGCGTATTGGG
 
GTCAAGGCACCATGGTGACCGTTAGCAGCAAAGCGTCAGGCGGTCCGGAAGGCGGT
TCGCTGGCAGCTCTGACCGCACATCAGGCATGCCACCTGCCGCTGGAAACCTTTACC
CGCCATCGTCAACCGCGCGGCTGGGAACAGCTGGAACAATGTGGTTATCCGGTGCAG
CGCCTGGTTGCCCTGTACCTGGCCGCACGTCTGAGCTGGAACCAGGTTGATCAAGTC
ATTCGTAATGCGCTGGCATCACCGGGCTCGGGCGGTGACCTGGGTGAAGCAATCCGC
GAACAGCCGGAACAAGCGCGCCTGGCCCTGACCCTGGCAGCTGCGGAATCCGAACG
CTTTGTGCGTCAGGGCACCGGTAATGATGAAGCCGGTGCAGCAAATGGTCCGGCTGA
TTCAGGTGACGCGCTGCTGGAACGCAACTATCCGACGGGCGCCGAATTTCTGGGTGA
TGGCGGTGACGTGAGTTTCTCCACCCGCGGCACGCAGAATTGGACCGTTGAACGTCT
GCTGCAGGCGCATCGCCAACTGGAAGAACGTGGTTATGTTTTTGTCGGCTATCATGGC
ACCTTTCTGGAAGCTGCGCAGTCGATTGTCTTTGGCGGTGTGCGTGCACGCAGCCAG
GATCTGGATGCAATTTGGCGCGGCTTCTACATCGCAGGTGATCCGGCTCTGGCGTATG
GCTACGCTCAGGATCAAGAACCGGATGCGCGTGGCCGCATCCGTAATGGTGCCCTGC
TGCGTGTGTATGTTCCGCGTTCATCGCTGCCGGGTTTTTACCGTACCTCTCTGACCCTG
GCGGCACCGGAAGCTGCCGGCGAAGTGGAACGCCTGATTGGTCACCCGCTGCCGCT
GCGTCTGGATGCAATCACCGGTCCGGAAGAAGAAGGCGGCCGTCTGGAAACGATTCT
GGGTTGGCCGCTGGCCGAACGTACCGTGGTTATTCCGAGCGCAATCCCGACGGACCC
GCGCAATGTTGGCGGTGATCTGGACCCGAGCTCTATTCCGGATAAAGAACAGGCCAT
CTCCGCACTGCCGGACTATGCGTCACAACCGGGCAAACCGCCGCGTGAAGACCTGA
AA
SEQ ID NO. 23: Version 5 VH-PE38 DNA sequence 
ATGGAAGTGCAGCTGGTTGAAAGCGGTGGCGGTCTGGTGAAACCGGGCGGTAGCCT
GCGTCTGAGCTGCGCGGCGAGCGGTTTCGCGTTTAGCATCTACGACATGAGCTGGATT
CGTCAAGCGCCGGGCAAATGCCTGGAGTGGGTGGCGTATATCAGCAGCGGCGGTGGC
ACCACCTACTATCCGGGTAGCGTTAAGGGCCGTTTCACCATTAGCCGTGAAAACGCG
AAAAACAGCCTGTACCTGCAGATGAACAGCCTGCGTTCTGAGGATACCGCTGTGTAC
TATTGCGCGCGTCACAGCGGTTACGGCACCCACTGGGGCGTTCTGTTTGCGTATTGGG
GTCAAGGCACCATGGTGACCGTTAGCAGCAAAGCGTCAGGCGGTCCGGAAGGCGGT
TCGCTGGCAGCTCTGACCGCACATCAGGCATGCCACCTGCCGCTGGAAACCTTTACC
CGCCATCGTCAACCGCGCGGCTGGGAACAGCTGGAACAATGTGGTTATCCGGTGCAG
 
CGCCTGGTTGCCCTGTACCTGGCCGCACGTCTGAGCTGGAACCAGGTTGATCAAGTC
ATTCGTAATGCGCTGGCATCACCGGGCTCGGGCGGTGACCTGGGTGAAGCAATCCGC
GAACAGCCGGAACAAGCGCGCCTGGCCCTGACCCTGGCAGCTGCGGAATCCGAACG
CTTTGTGCGTCAGGGCACCGGTAATGATGAAGCCGGTGCAGCAAATGGTCCGGCTGA
TTCAGGTGACGCGCTGCTGGAACGCAACTATCCGACGGGCGCCGAATTTCTGGGTGA
TGGCGGTGACGTGAGTTTCTCCACCCGCGGCACGCAGAATTGGACCGTTGAACGTCT
GCTGCAGGCGCATCGCCAACTGGAAGAACGTGGTTATGTTTTTGTCGGCTATCATGGC
ACCTTTCTGGAAGCTGCGCAGTCGATTGTCTTTGGCGGTGTGCGTGCACGCAGCCAG
GATCTGGATGCAATTTGGCGCGGCTTCTACATCGCAGGTGATCCGGCTCTGGCGTATG
GCTACGCTCAGGATCAAGAACCGGATGCGCGTGGCCGCATCCGTAATGGTGCCCTGC
TGCGTGTGTATGTTCCGCGTTCATCGCTGCCGGGTTTTTACCGTACCTCTCTGACCCTG
GCGGCACCGGAAGCTGCCGGCGAAGTGGAACGCCTGATTGGTCACCCGCTGCCGCT
GCGTCTGGATGCAATCACCGGTCCGGAAGAAGAAGGCGGCCGTCTGGAAACGATTCT
GGGTTGGCCGCTGGCCGAACGTACCGTGGTTATTCCGAGCGCAATCCCGACGGACCC
GCGCAATGTTGGCGGTGATCTGGACCCGAGCTCTATTCCGGATAAAGAACAGGCCAT
CTCCGCACTGCCGGACTATGCGTCACAACCGGGCAAACCGCCGCGTGAAGACCTGA
AA
SEQ ID NO. 24: Version 1 VL DNA sequence 
ATGGACATCCAGATGACCCAGTCTCCTTCTACACTGTCTGCTTCTGTGGGCGACAGAG
TGACCATCACCTGCAGAGCCTCTCAGGACATCTCCAACTACCTGAACTGGTACCAGC
AGAGACCTGGCCAGTCCGTGAAGCTGCTGATCTACTACACCTCCATCCTGCACTCCG
GCGTGCCTTCCAGATTCTCCGGCTCTGGATCTGGCACCGACTACACCCTGACCATCTC
CTCCCTGCAGCCTGAGGACTTCGCCACCTACTTCTGCCAGCAGGGCAACACCCTGCC
TTGGACCTTCGGCTGCGGCACCAAGGTGGAAATCAAG
SEQ ID NO. 25: Version 2 VL DNA sequence 
ATGGACATCCAGATGACCCAGTCTACTTCTACACTGTCTGCTTCTGTGGGCGACAGAG
TGACCATCACCTGCAGAGCCTCTCAGGACATCTCCAACTACCTGAACTGGTACCAGC
AGAGACCTGATGGCTCCGTGAAGCTGCTGATCTACTACACCTCCATCCTGCACTCCGG
CGTGCCTTCCAGATTCTCCGGCTCTGGATCTGGCACCGACTACACCCTGACCATCTCC
 
TCCCTGCAGGATGAGGACTTCGCCACCTACTTCTGCCAGCAGGGCAACACCCTGCCT
TGGACCTTCGGCTGCGGCACCAAGGTGGAAATCAAG
SEQ ID NO. 26: Version 3 VL DNA sequence 
ATGGACATCCAGATGACCCAGAGCCCGAGCACCCTGAGCGCGAGCGTGGGCGACCG
TGTTACCATCACCTGCCGTGCGAGCCAGGATATTAGCAACTACCTGAACTGGTTTCAA
CAACGTCCGGGTCAAAGCCCGCGTCGTCTGATCTACTATACCAGCATTCTGCACAGCG
GTGTGCCGAGCCGTTTTAGCGGTAGCGGCAGCGGTACCGACTATACCCTGACCATCA
GCAGCCTGCAGCCGGAGGATTTCGCGACCTACTATTGCCAGCAGGGTAACACCCTGC
CGTGGACCTTTGGCTGCGGTACCAAGGTTGAAATTAAA
SEQ ID NO. 27: Version 3 VL DNA sequence 
ATGGACATCCAGATGACCCAGAGCCCGAGCACCCTGAGCGCGAGCGTGGGCGACCG
TGTTACCATCACCTGCCGTGCGAGCCAGGATATTAGCAACTACCTGAACTGGTTTCAA
CAACGTCCGGGTCAAAGCCCGCGTctgCTGATCTACTATACCAGCATTCTGCACAGCGG
TGTGCCGAGCCGTTTTAGCGGTAGCGGCAGCGGTACCGACTATACCCTGACCATCAGC
AGCCTGCAGCCGGAGGATTTCGCGACCTACTATTGCCAGCAGGGTAACACCCTGCCG
TGGACCTTTGGCTGCGGTACCAAGGTTGAAATTAAA
SEQ ID NO. 28: HA22, H-CDR1
   GFAFSIYD
SEQ ID NO. 29: HA22, H-CDR2
   ISSGGGTTY
SEQ ID NO. 30: HA22, H-CDR3
   CARHSGYGTHWGVLFAY
SEQ ID NO. 31: HA22, L-CDR1
   QDISNY
SEQ ID NO. 32: HA22, L-CDR2
   YTSILHSG
SEQ ID NO. 33: HA22, L-CDR3
   QQGNTLP

The present invention is illustrated below with reference to specific examples. It will be understood by those skilled in the art that these examples are merely illustrative of the present invention and do not limit the scope of the present invention in any way.

Experimental procedures in the following examples are all conventional ones, unless otherwise specified. Raw materials and reagents used in the following examples are all commercially available products, unless otherwise specified.

### Example 1: Humanization design

A framework shuffling strategy was used to alter amino acids in the framework regions of heavy chain variable region (V_{H}, SEQ ID NO. 2) and light chain variable region (V_{L}, SEQ ID NO. 14) from the original murine to human ones. All the CDRs, which were determined using both the Kabat and Chothia numbering systems and the online tool (http://www.bioinf.org.uk/abs/), were left unchanged. Each of the framework and J regions in both the VH and VL of HA22 was aligned to the human antibody germline sequences in the international ImMunoGeneTics information system^{®} (IMGT: http://www.imgt.org) for the closest human antibody matches. Murine residues that were deemed Vernier residues or important for VH/VL interactions were not changed^{[26]} to preserve the specificity and affinity towards CD22.

A panel of humanization designs were prepared and evaluated to select the final candidate that possessed maximum humanization while maintaining optimal affinity and efficacy profile. The updated VH and VL formed a disulfide-linked Fv portion of anti-CD22 antibody fused to a Pseudomonas exotoxin A (PE38, SEQ ID NO. 1) (Figure 1).

The alignment revealed a combination of human germline sequences framework 1 of IGHV3-15*01, framework 2 of IGHV3-11*04, framework 3 of IGHV3-13*01, and framework 4 of IGHJ3*02 would provide humanized frameworks most closely resembling those of VH of HA22; and similarly a combination of framework 1 of IGKV1-5^{∗}01, framework 2 of IGKV2D-30*01, framework 3 of IGKV1D-43^{∗}01, and framework 4 of IGKJ1^{∗}01 would provide humanized frameworks most closely resembling those of VL of HA22. The Vernier residues were reverted to the original murine residues after rounds of iterations to show that they were critical for antigen bindings.

The sequence alignment provided in Figure 2 shows examples of the humanization process. In the alignment, HA22VH and HA22VL are the Fv fragments used in HA22. Version 3 (V3) is the fully humanized VH and VL versions. Version 1, 2, 4, and 5 (Vl, V2, V4, and V5) are different versions of designs that have critical residues reverted to murine ones to restore lost activities. Residues shaded are Vernier residues. Residues underlined are in CDRs based on IMGT criteria.

### Example 2: Production of the humanized anti-CD22 recombinant immunotoxin

Expression plasmids capable of expressing different VL humanized versions (the first polypeptide chains) were constructed. In addition, 5' terminus of the nucleotide sequence coding for PE38 was linked to 3' terminus of the nucleotide sequence coding for each of the different VH humanized versions respectively to form a single nucleotide chain, with which expression plasmids capable of expressing the second polypeptide chains were constructed. Schematic diagrams are shown in Figure 3 (synthesized and constructed by Genscript according to the designs).

The humanized anti-CD22 recombinant immunotoxins provided in the present disclosure were produced in an enhanced BL21 derivative strain (T7 Express) using the expression plasmids of the first and second polypeptide chains.

### 1. Fermentation

The first polypeptide chain and second polypeptide chain seed cultures were prepared, respectively, in a fermentation medium containing 30 g soytone, 30 g yeast extract, 40 g glycerol, 2 g NH₄Cl, 2 g (NH₄)₂SO₄, 0.973 g MgSO₄, 3 g glucose, and 1 g NaCl per L. The seed cultures were incubated at 37 °C, 250 RPM for 12-16 hours. The fermentation runs for the first polypeptide chain and second polypeptide chain were performed using a New Brunswick BioFlo 3000 Fermentor. The fermentor was inoculated at 2% with the respective first polypeptide chain and second polypeptide chain seed. The pH was set at 6.9 and controlled during the run by addition of ammonia (NH₃•H₂O). The dissolved oxygen (DO) was set at 30% and controlled by agitation, aeration and oxygen supplementation. The vessel pressure was set at 2 psi. The cell density (OD600) and glucose concentrations, together with pH, DO, and temperature, were monitored hourly during the run. Four hours post inoculation, at OD600 8-9, the glucose in the medium was depleted and additional glucose was fed into the fermentor. The glucose feeding lasted for an hour, with a total volume of 1.7% of original medium volume. The additional glucose was exhausted at around 5.5 hours after inoculation. 0.1% volume of 1 M IPTG was then added to start the induction/expression phase. During the induction stage, pH was allowed to increase gradually to 7.0. Three hours post induction, the cells were harvested by centrifugation at 8,000 × g, at 4 °C, for 20 minutes to remove the medium. The cell paste was stored at -80 °C before processing.

### 2. Recovery and refolding

The first polypeptide chain and second polypeptide chain inclusion bodies were isolated by disrupting the cells using a high-pressure homogenizer. The lysate pellet containing the inclusion body was then purified through several washing steps with centrifugation and detergent to remove cell debris and soluble impurities.

The washed inclusion bodies were resuspended in a solubilization buffer (50 mM ethanolamine, 8 M Urea, 0.5 M arginine, 2 mM EDTA, 10 mM DTT, pH 9.3) containing a strong denaturant (8 M urea) and a reducing agent (10 mM DTT) to keep all cysteines in the reduced state and cleave disulfide bonds formed during the preparation. Solubilization was performed at 2-8 °C with stirring to facilitate the solubilization process for > 2 hours. After solubilization, the inclusion body solution was centrifuged at 13,000 × g for 30 minutes and the supernatant containing the solubilized inclusion body was filtered to remove the remaining aggregates and insoluble impurities.

Total protein concentrations were determined based on A280 reading and percentages of the first polypeptide chain and second polypeptide chain were estimated by densitometry analysis of SDS-PAGE gel. The first polypeptide chain and second polypeptide chain were then mixed at 1:1 molar ratio, diluted to 10 mg/mL with solubilization buffer, and fed into the refolding buffer (50 mM ethanolamine, 0.5 M arginine, 2 mM EDTA, 0.9 mM oxidized glutathione, pH 9.4) at a 1:10 ratio continuously over a 24-hour period and incubated at 2-8 °C for an additional 48-72 hours. The refolding reaction was stopped by adjusting the pH to ∼ 7.40. The refolded protein was then concentrated and prepared for column purification by tangential flow filtration.

### 3. Phenyl HP hydrophobic interaction chromatography

Phenyl HP hydrophobic interaction chromatography was carried out in a chromatography column with 17 cm bench height (GE Healthcare, Marlborough, MA USA). All runs were conducted using an AKTA avant liquid chromatography system from GE Healthcare (Marlborough, MA USA) and the column was operated at 95 cm/hr. The column was equilibrated with 20 mM Tris-HCl, 0.6 M Na₂SO₄, pH 7.4. Load was prepared by diluting 1 part (by weight) protein solution with 1 part 20 mM Tris-HCl, 1.2 M Na₂SO₄, pH 7.4. After loading, the column was washed with equilibration buffer and then eluted in a linear gradient of Na₂SO₄ from 0.6 M to 0 M over 16 column volumes. The product peak was collected in fractions.

### 4. POROS HQ anion exchange chromatography

POROS HQ anion exchange chromatography was carried out in a chromatography column packed to 30 cm bed height (Thermo Scientific, Waltham, MA USA). All runs were conducted using an AKTA avant liquid chromatography system from GE Healthcare and the column was operated at 275 cm/hr. The column was equilibrated with 20 mM Tris-HCl, pH 7.4, loaded with protein, and then washed with equilibration buffer. The column was eluted in a linear gradient of NaCl from 0 M to 0.5 M over 25 column volumes. The product peak was collected based on absorbance criteria of 5 mAU on the leading and tailing side of the product peak.

### 5. Capto Q anion exchange chromatography

Capto Q anion exchange chromatography was carried out in flow-through mode in a chromatography column packed to 10 cm bed height (GE Healthcare, Marlborough, MA USA).

All runs were conducted using an AKTA avant liquid chromatography system from GE Healthcare and the column was operated at 250-350 cm/hr. The column was equilibrated with 20 mM Tris-HCl, 250 mM NaCl, pH 7.4, loaded with protein, and then washed with equilibration buffer. The product peak was collected based on absorbance criteria of 5 mAU on the leading and tailing side of the product flow-through peak.

### Example 3: Non-clinical pharmacology of the humanized anti-CD22 recombinant immunotoxins

As described in Example 2, different recombinant immunotoxins were prepared with different VH and VL humanized versions provided in the present disclosure, as well as with the VH and VL of HA22, and were named following a format "HmLn", in which m and n is the number of the VH and VL humanized versions.

### 3.1 Target interaction

ELISA was performed with recombinant human Siglec-2/CD22 Fc chimera protein (R&D systems, Cat# 1968-SL) as the immobilized antigen, which captures the humanized anti-CD22 recombinant immunotoxins or HA22 molecule provided in the present disclosure, and anti-Pseudomonas Exotoxin A antibody (Sigma-Aldrich, Cat# P2318) as the secondary antibody containing the enzyme conjugate for detection.

Octet assay was performed with streptavidin (SA) biosensors (ForteBio, Cat# 18-5019) conjugated with biotinylated CD22 for the K_{D} measurement of the recombinant immunotoxins provided in the present disclosure or HA22 molecule.

As shown in Table 1, recombinant immunotoxins H1L2 (containing V_{H} ver 1 + V_{L} ver 2) and H2L2 (containing V_{H} ver 2 + V_{L} ver 2) showed comparable EC₅₀ and K_{D} to HA22.

**Table 1. CD22 binding assay results**

| Sample | EC₅₀ by ELISA | K_{D} by Octet |
|---|---|---|
| HA22 | 0.47 ± 0.48 nM | 4.08 - 5.81 nM |
| H1L2 | 0.54 ± 0.24 nM | 4.66 - 7.21 nM |
| H2L2 | 0.56 ± 0.33 nM | 5.01 - 7.02 nM |

Different versions of the humanized recombinant immunotoxins were analyzed for CD22 binding affinity, as shown in Table 2 and Table 3. In Table 2, a range of EC₅₀ by ELISA is given or presented as Mean ± SD when two or no less than three batches of products were assayed in separate analyses, respectively. Results of EC₅₀ of the humanized recombinant immunotoxins relative to HA22 are shown in Table 3.

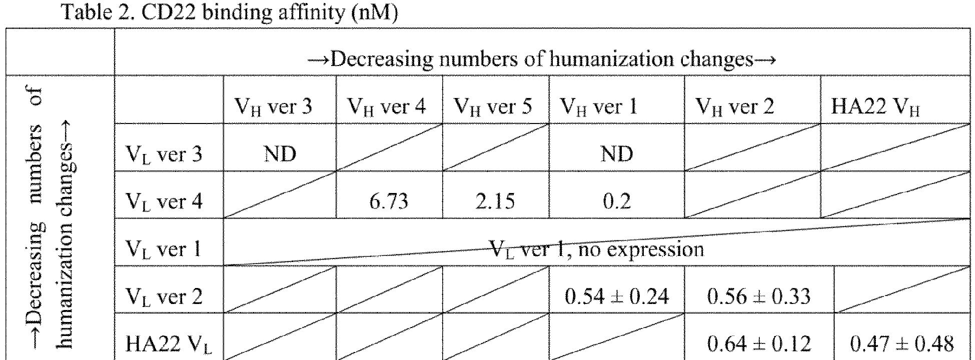

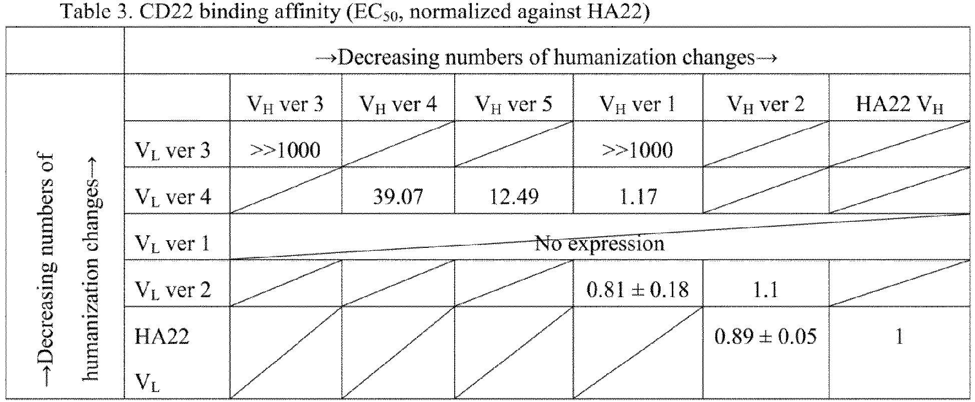

### 3.2 Cell-based potency assay

A CD22-expressing cell model was employed in cell-based assay to evaluate the growth inhibition and apoptotic cell death effects of the humanization anti-CD22 recombinant immunotoxins in the present disclosure. This is an endpoint assay using cell growth/inhibition as the indicator of potency. The cell model better mimics the in vivo condition comparing to the binding assay, in which, the CD22 binding, internalization, and ADP ribosylation of EF-2 catalyzed by PE38 are all involved to actuate the observed inhibitory effect.

In this assay, the Daudi cell line expressing a high level of CD22, was co-cultured with the recombinant immunotoxins or HA22 molecule provided in the present disclosure for a certain period of time and, at the end of the assay, live cells in the culture were quantified with a colorimetric assay. The procedure is as follows.

Daudi cells described above were cultured in RPMI-1640 complete medium, and harvested and washed with HBSS once. The cells were resuspended in RPMI-1640 medium at a density of 2.5×10⁵/mL, and transferred to a 96-well plate at 100 µL/well (equivalent to 2.5×10⁴ live cells per well). The recombinant immunotoxins or HA22 provided in the present disclosure to be detected were diluted to predetermined concentrations and serial dilutions were prepared.

100 µL of each dilution was added to the cells in the wells, and the cells were incubated at 37 °C, 5% CO₂ for 48-72 hours. Afterwards, 10 µL of CCK-8 agent was added into each well of the plate which was then incubated at 37 °C for 4-8 hours. To correct for background activity, the cells were cultured in the presence of 10 µg/mL cycloheximide until 100% of the cells died.

The generation of Formazan was measured by detecting the absorbance at 450 nm. Values were normalized against cycloheximide and medium control, and the concentration of each of the humanized recombinant immunotoxins and HA22 provided in the present disclosure when 50% of the cells died was measured as EC₅₀.

Different versions of the humanized recombinant immunotoxins were analyzed for CD22 binding affinity, as shown in Table 4 and Table 5. In Table 4, a range of EC₅₀ is given or presented as Mean ± SD when two or no less than three batches of products were assayed in separate analyses, respectively. Results of EC₅₀ of the humanized recombinant immunotoxins relative to HA22 are shown in Table 5. As shown, the humanized recombinant immunotoxins, especially H1L2 showed comparable EC₅₀ to HA22 in *in vitro* cell-based potency assay.

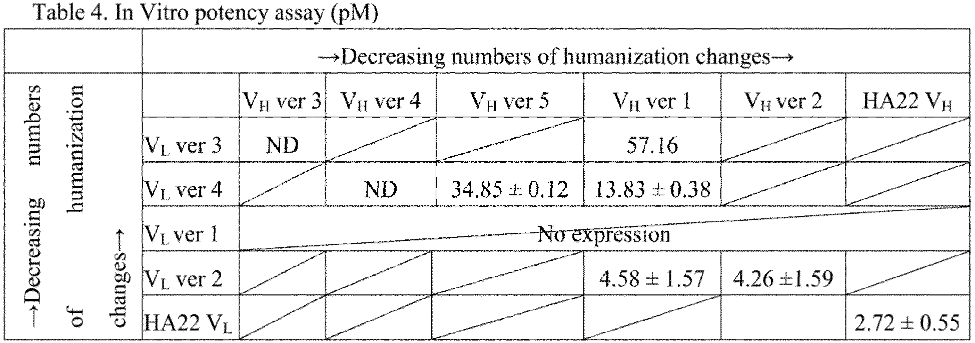

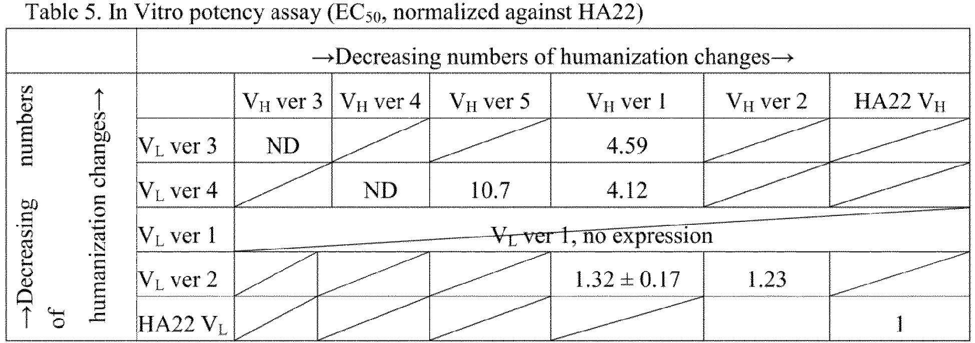

### Example 4: Non-clinical pharmacokinetics and drug metabolism of the humanized anti-CD22 recombinant immunotoxins

### 4.1 In vivo PK studies

A C57BL16 mouse model (4-6 week-old, female) was used in this study. 45 mice were randomly divided into 3 groups: 1) control (formulation buffer), 2) 500 µg/kg HA22; and 3) 500 µg/kg H1L2. The control or treatment groups were given via tail vein injection, and 100 µL total blood was collected at specified time points after injection (5 min, 15 min, 30 min, 45 min, 1 h, 2 h, 4 h, 8 h, 24 h, 48 h, 96 h) via retro orbital sampling. Three replicate animals were assigned to each time point. 50 µL plasma was recovered from the blood sample for analysis.

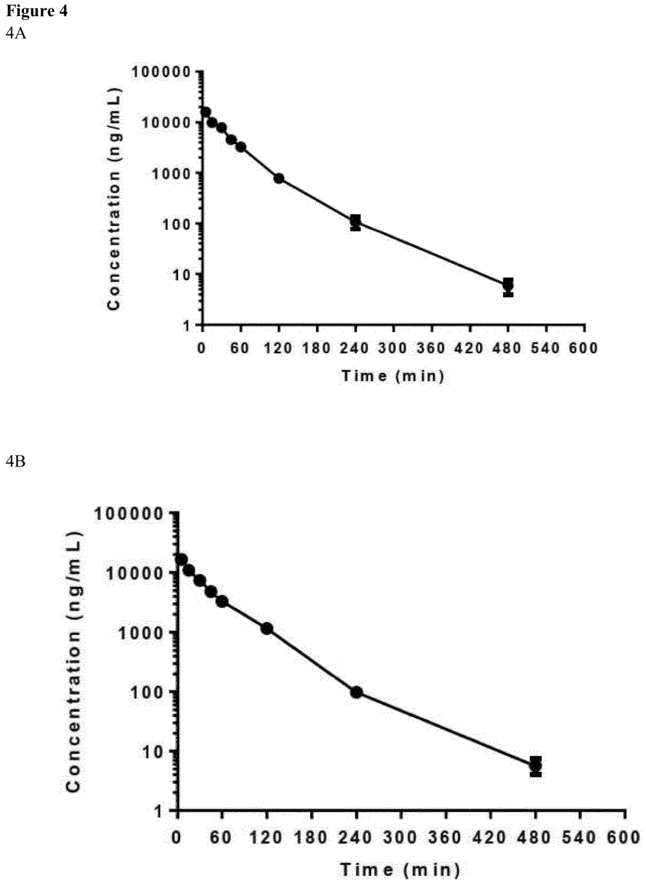

As shown in Table 6 and Table 7, results showed that in the mouse model H1L2 showed comparable pharmacokinetics properties to those of HA22: both molecules exhibited similar half-life (T_{1/2}), time to reach maximum concentration (Tₘₐₓ), maximum concentration (Cₘₐₓ) as well as area under the curve (AUC₀₋ₜ). PK profile is shown in Figure 4.

**Table 6. HA22 PK result**

| | T_{1/2} (min) | Tₘₐₓ (min) | Cₘₐₓ (ng/mL) | AUC₀₋ₜ, (ng/mL×min) |
|---|---|---|---|---|
| Replicate 1 | 44.38 | 5 | 1.671×10⁴ | 7.329×10⁵ |
| Replicate 2 | 46.32 | 5 | 1.275×10⁴ | 6.385×10⁵ |
| Replicate 3 | 57.26 | 5 | 1.863×10⁴ | 7.125×10⁵ |
| Average | 49.32 | 5 | 1.603×10⁴ | 6.947×10⁵ |

**Table 7. H1L2PK result**

| | T_{1/2} (min) | Tₘₐₓ (min) | Cₘₐₓ (ng/mL) | AUC₀₋ₜ (ng/mL×min) |
|---|---|---|---|---|
| Replicate 1 | 41.69 | 5 | 1.906×10⁴ | 7.416×10⁵ |
| Replicate 2 | 41.76 | 5 | 1.581×10⁴ | 7.458×10⁵ |
| Replicate 3 | 42.48 | 5 | 1.910×10⁴ | 7.474×10⁵ |
| Average | 41.98 | 5 | 1.799×10⁴ | 7.449×10⁵ |

### 4.2 In vivo efficacy

A xenograft animal model and the CD22-positive Raji B lymphocytes were employed for tumor establishment. Upon administration, the recombinant immunotoxin molecule enters the circulation, binds to the CD22 on the surface of the malignant B-cells, and suppresses the cell growth via the apoptotic pathways. Therefore, the inhibition of tumor growth was designated as the endpoint of the efficacy study in this Example.

An athymic NCr nude mouse model (4-6 week-old, female) was used. 25 mice were randomly divided into 5 groups as shown in Table 8. A CD-22 positive Homo sapiens lymphoblast Raji cell line (ATCC CCL-86) was used to establish the xenograft. Raji cells were subcutaneously injected at a final concentration of 5 × 10⁷ cells/mL and grown to 100-200 mm³ before any treatment. Once the xenograft tumor was established, a single dose of the treatments at the indicated dose level was given via tail vein injection. The xenograft mice were observed and measured for tumor size for 24 days after the treatment.

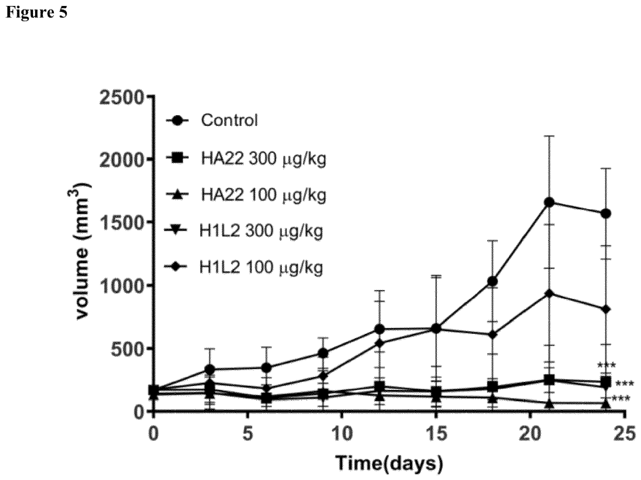

As shown in Figure 5, comparing to the control group (the first group), HA22 high dose group showed 84.9% suppression of tumor growth, low dose group showed 95.8% suppression of tumor growth; and, H1L2 high dose group showed 87.7% suppression of tumor growth, low dose group showed 34.2% suppression of tumor growth. A T test was performed, and all treatments except for the humanized recombinant immunotoxin H1L2 at low dose showed significant tumor suppression efficacy (in Figure 5, * *P* < 0.05, ** *P* < 0.01, *** *P* < 0.005). There were no statistic differences between the high dose groups of HA22 and H1L2.

**Table 8. Efficacy study group and dosing information**

| Group | Treatment | Dose level (µg/kg) | No. of animals |
|---|---|---|---|
| 1 | Formulation buffer | 0 | 5 |
| 2 | HA22 | 100 | 5 |
| 3 | HA22 | 300 | 5 |
| 4 | H1L2 | 100 | 5 |
| 5 | H1L2 | 300 | 5 |

The above description of the embodiments of the present invention is not intended to limit the present invention, and those skilled in the art may make various changes and modifications to the present invention without departing from the spirit of the present invention, which should fall within the scope of the appended claims.

### References:

1. Macauley, M.S., P.R. Crocker and J.C. Paulson, Siglec-mediated regulation of immune cell function in disease. Nature Reviews Immunology, 2014. 14(10): p. 653.
2. Naeim, F., Atlas of hematopathology: morphology, immunophenotype, cytogenetics, and molecular approaches. 2012: Academic Press. Ch. 2, p. 33.
3. Kreitman, R.J. and E. Arons, Update on hairy cell leukemia. Clinical advances in hematology & oncology: H&O, 2018.16(3): p. 205.
4. Grever, M.R. et al., Consensus guidelines for the diagnosis and management of patients with classic hairy cell leukemia. Blood, 2017. 129(5): p. 553-60.
5. Holzman, D., Has success spoiled hairy cell leukemia research? Key questions go unanswered, despite big gains. 2009, Oxford University Press.
6. Grever, M.R. et al., Consensus guidelines for the diagnosis and management of patients with classic hairy cell leukemia. Blood, 2017. 129(5): p. 553-60.
7. Getta, B.M., J.H. Park and M.S. Tallman, Hairy cell leukemia: past, present and future. Best Practice & Research Clinical Haematology, 2015. 28(4): p. 269-72.
8. Kreitman, R.J., Immunoconjugates and new molecular targets in hairy cell leukemia. ASH Education Program Book, 2012. 2012(1): p. 660-6.
9. Pastan, I., Immunotoxins containing Pseudomonas exotoxin A: a short history. Cancer Immunology, Immunotherapy, 2003. 52(5): p. 338-41.
10. Rust, A. et al., The Use of Plant-Derived Ribosome Inactivating Proteins in Immunotoxin Development: Past, Present and Future Generations. Toxins (Basel), 2017. 9(11): p.344.
11. Sievers, E.L. and P.D. Senter, Antibody-drug conjugates in cancer therapy. Annu Rev Med, 2013.64: p. 15-29.
12. Mansfield, E., 1. Pastan and D.J. FitzGerald, Characterization of RFB4- Pseudomonas Exotoxin A Immunotoxins Targeted to CD22 on B-Cell Malignancies. Bioconjugate chemistry, 1996. 7(5): p. 557-63.
13. Mansfield, E. et al., Recombinant RFB4 immunotoxins exhibit potent cytotoxic activity for CD22-bearing cells and tumors. Blood, 1997. 90(5): p. 2020-6.
14. Kreitman, R.J. et al., Efficacy of the anti-CD22 recombinant immunotoxin BL22 in chemotherapy-resistant hairy-cell leukemia. New England Journal of Medicine, 2001. 345(4): p. 241-7.
15. Kreitman, R.J. et al., Phase I trial of recombinant immunotoxin RFB4 (dsFv)-PE38 (BL22) in patients with B-cell malignancies. J Clin Oncol, 2005. 23(27): p. 6719-29.
16. Wayne, A.S. et al., Anti-CD22 Immunotoxin RFB4(dsFv)-PE38 (BL22) for CD22-Positive Hematologic Malignancies of Childhood: Preclinical Studies and Phase 1 Clinical Trial. Clinical Cancer Research, 2010: p. 1078-0432.CCR-09-2980.
17. Mussai, F. et al., Cytotoxicity of the anti - CD22 immunotoxin HA22 (CAT - 8015) against paediatric acute lymphoblastic leukaemia. British journal of haematology, 2010. 150(3): p. 352-358.
18. Wayne, A.S. et al., Immunotoxins for leukemia. Blood, 2014. 123(16): p. 2470-7.
19. Alewine, C., R. Hassan and I. Pastan, Advances in anticancer immunotoxin therapy. Oncologist, 2015. 20(2): p. 176-85.
20. Kreitman et al., Moxetumomab pasudotox in relapsed/refractory hairy cell leukemia, Leukemia. 2018; 32(8): 1768 - 1777.
21. Mazor, R. et al., Elimination of murine and human T-cell epitopes in recombinant immunotoxin eliminates neutralizing and anti-drug antibodies in vivo. Cell Mol Immunol 14, 432 -442 (2017).
22. Liu, W. et al., Recombinant immunotoxin engineered for low immunogenicity and antigenicity by identifying and silencing human B-cell epitopes. Proc. Natl. Acad. Sci. USA 2012, 109, 11782 - 11787.
23. Mazor, R. et al., Recombinant immunotoxin for cancer treatment with low immunogenicity by identification and silencing of human T-cell epitopes. Proc. Natl. Acad. Sci. USA 2014, 111, 8571 - 8576.
24. Mazor, R. et al., Recombinant immunotoxin with T-cell epitope mutations that greatly reduce immunogenicity for Treatment of mesothelin-expressing tumors. Mol. Cancer Ther. 2015, 14, 2789 - 2796.
25. Moss, D.L. et al., Deimmunizing substitutions in Pseudomonas exotoxin domain III perturb antigen processing without eliminating T-cell epitopes. J. Biol. Chem. 2019, 294, 4667-4681.
26. Varkey, R. et al., Discovery and characterization of potent IL-21 neutralizing antibodies via a novel alternating antigen immunization and humanization strategy. PLOS ONE, 2019. 14(1): p. e0211236.

## Claims

1. A humanized anti-CD22 recombinant immunotoxin which is a polypeptide molecule comprising two polypeptide chains as follows:
(1) a first polypeptide chain, comprising a light chain variable region (VL) of an anti-CD22 antibody, wherein the light chain variable region (VL) comprises an amino acid sequence as shown in SEQ ID NO. 16 or SEQ ID NO. 18 or an amino acid sequence homologue thereof having at least 75% sequence identity to the amino acid sequence as shown in SEQ ID NO. 16 or SEQ ID NO. 18;
(2) a second polypeptide chain, comprising a heavy chain variable region (V_{H}) of an anti-CD22 antibody and a cytotoxin directly or indirectly linked to the heavy chain variable region (V_{H}), wherein the heavy chain variable region (V_{H}) comprises an amino acid sequence as shown in SEQ ID NO. 3 or SEQ ID NO. 4 or an amino acid sequence homologue thereof having at least 75% identity to the amino acid sequence as shown in SEQ ID NO. 3 or SEQ ID NO. 4.

2. The humanized anti-CD22 recombinant immunotoxin according to claim 1, wherein the amino acid sequence homologue in the first polypeptide chain has at least 80%, at least 85%, at least 90%, at least 91%, at least 92%, at least 93%, at least 94%, at least 95%, at least 96%, at least 97%, at least 98%, or at least 99% sequence identity to the amino acid sequence as shown in SEQ ID NO. 16 or SEQ ID NO. 18; preferably, the amino acid sequence homologue comprises the amino acid sequences as shown in SEQ ID NO. 31, SEQ ID NO. 32 and SEQ ID NO. 33, and has the same amino acid residues as those in SEQ ID NO. 16 or SEQ ID NO. 18 at positions 3, 5, 36, 37, 47, 48, 49, 50, 65, 67, 69, 70 and 72 corresponding to the amino acid sequence as shown in SEQ ID NO. 14, but is not the amino acid sequence as shown in SEQ ID NO. 14;
preferably, the amino acid sequence homologue in the second polypeptide chain has at least 80%, at least 85%, at least 90%, at least 91%, at least 92%, at least 93%, at least 94%, at least 95%, at least 96%, at least 97%, at least 98%, or at least 99% sequence identity to the amino acid sequence as shown in SEQ ID NO. 3 or SEQ ID NO. 4; preferably, the amino acid sequence homologue comprises the amino acid sequences as shown in SEQ ID NO. 28, SEQ ID NO. 29 and SEQ ID NO. 30, and has the same amino acid residues as those in SEQ ID NO. 3 or SEQ ID NO. 4 at positions 3, 48, 49, 50, 69, 71, 73, 75, and 80 corresponding to the amino acid sequence as shown in SEQ ID NO. 2, but is not the amino acid sequence as shown in SEQ ID NO. 2.

3. The humanized anti-CD22 recombinant immunotoxin according to claim 1 or 2, wherein the light chain variable region (V_{L}) in the first polypeptide chain comprises the amino acid sequence as shown in SEQ ID NO. 16 or the amino acid sequence homologue thereof; and the heavy chain variable region (V_{H}) in the second polypeptide chain comprises the amino acid sequence as shown in SEQ ID NO. 3 or the amino acid sequence homologue thereof; or
the light chain variable region (V_{L}) in the first polypeptide chain comprises the amino acid sequence as shown in SEQ ID NO. 18 or the amino acid sequence homologue thereof; and the heavy chain variable region (VH) in the second polypeptide chain comprises the amino acid sequence as shown in SEQ ID NO. 3 or the amino acid sequence homologue thereof; or
the light chain variable region (V_{L}) in the first polypeptide chain comprises the amino acid sequence as shown in SEQ ID NO. 16 or the amino acid sequence homologue thereof; and the heavy chain variable region (V_{H}) in the second polypeptide chain comprises the amino acid sequence as shown in SEQ ID NO. 4 or the amino acid sequence homologue thereof;
preferably, in the recombinant immunotoxin, the light chain variable region (V_{L}) in the first polypeptide chain and the heavy chain variable region (V_{H}) in the second polypeptide chain are covalently linked, e.g., via a disulfide bond;
preferably, the cytotoxin in the second polypeptide chain is pseudomonas exotoxin A or a mutant or fragment of the pseudomonas exotoxin A with cytotoxicity retained; preferably, the mutant or fragment of the pseudomonas exotoxin A is PE40, PE38, PE35, PE24, mPE24, T19, T20, or M11.

4. The humanized anti-CD22 recombinant immunotoxin according to any one of claims 1 to 3, wherein in the second polypeptide chain, the cytotoxin is fused to the C-terminus of the heavy chain variable region (V_{H}) of the anti-CD22 antibody;
preferably, the N-terminus of the cytotoxin is fused, directly or via a linker, to the C-terminus of the heavy chain variable region (V_{H});
preferably, the first polypeptide chain further comprises a kappa light chain constant region, preferably a human kappa light chain constant region fused to the C-terminus of the light chain variable region (V_{L}); alternatively, the second polypeptide chain further comprises a heavy chain constant region CH1, preferably a human heavy chain constant region CH1 fused to the C-terminus of the heavy chain variable region (V_{H}).

5. The humanized anti-CD22 recombinant immunotoxin according to any one of claims 1 to 4, wherein the first polypeptide chain comprises the amino acid sequence as shown in SEQ ID NO. 16 or the amino acid sequence homologue thereof, and the second polypeptide chain comprises the amino acid sequence as shown in SEQ ID NO. 9 or the amino acid sequence homolog thereof; or
the first polypeptide chain comprises the amino acid sequence as shown in SEQ ID NO. 18 or the amino acid sequence homologue thereof, and the second polypeptide chain comprises the amino acid sequence as shown in SEQ ID NO. 9 or the amino acid sequence homologue thereof; or
the first polypeptide chain comprises the amino acid sequence as shown in SEQ ID NO. 16 or the amino acid sequence homologue thereof, and the second polypeptide chain comprises the amino acid sequence as shown in SEQ ID NO. 10 or the amino acid sequence homologue thereof.

6. A nucleic acid molecule comprising a nucleotide sequence encoding the first polypeptide chain in the humanized anti-CD22 recombinant immunotoxin according to any one of claims 1 to 5 and/or a nucleotide sequence encoding the second polypeptide chain in the humanized anti-CD22 recombinant immunotoxin according to any one of claims 1 to 5.

7. A vector comprising the nucleic acid molecule according to claim 6.

8. A host cell transformed or transfected with the nucleic acid molecule according to claim 6 or the vector according to claim 7.

9. A composition comprising the humanized anti-CD22 recombinant immunotoxin according to any one of claims 1 to 5.

10. Use of the humanized anti-CD22 recombinant immunotoxin according to any one of claims 1 to 5, the nucleic acid molecule according to claim 6, the vector according to claim 7, the host cell according to claim 8 or the composition according to claim 9 in the manufacture of a medicament for the treatment of a CD22-related B cell malignant tumor;
preferably, the CD22-related B-cell malignant tumor is a B-cell malignant tumor **characterized by** high CD22 expression, such as a lymphoma or leukemia with high CD22 expression; more preferably, the lymphoma is non-Hodgkin's lymphoma, small lymphocytic lymphoma or mantle cell lymphoma; and the leukemia is chronic lymphocytic leukemia, hairy cell leukemia or acute lymphocytic leukemia.

11. A method for treating a CD22-related B-cell malignant tumor, comprising administering to a subject in need thereof the humanized anti-CD22 recombinant immunotoxin according to any one of claims 1 to 5, the nucleic acid molecule according to claim 6, the vector according to claim 7, the host cell according to claim 8 or the composition according to claim 9;
preferably, the CD22-related B-cell malignant tumor is a B-cell malignant tumor **characterized by** high CD22 expression, such as a lymphoma or leukemia with high CD22 expression; more preferably, the lymphoma is non-Hodgkin's lymphoma, small lymphocytic lymphoma or mantle cell lymphoma; and the leukemia is chronic lymphocytic leukemia, hairy cell leukemia or acute lymphocytic leukemia;
preferably, the subject is a mammal, including human or non-human primate, and a domestic, livestock or laboratory mammal such as a dog, a cat, a cow, a pig, a sheep, a horse, a murine, and a rabbit; more preferably, the subject is a human.

12. A humanized anti-CD22 antibody or antibody fragment, the antibody or antibody fragment comprising a heavy chain variable region (V_{H}) and a light chain variable region (V_{L}), wherein the heavy chain variable region (V_{H}) comprises an amino acid sequence as shown in SEQ ID NO. 3 or SEQ ID NO. 4 or an amino acid sequence homologue thereof having at least 75% identity to the amino acid sequence as shown in SEQ ID NO. 3 or SEQ ID NO. 4, and the light chain variable region (V_{L}) comprises an amino acid sequence as shown in SEQ ID NO. 16 or SEQ ID NO. 18 or an amino acid sequence homologue thereof having at least 75% identity to the amino acid sequence as shown in SEQ ID NO. 16 or SEQ ID NO. 18.
